(19) Europäisches Patentamt
European Patent Office
Office européen des brevets

(11) EP 0 979 827 A1

(12) EUROPÄISCHE PATENTANMELDUNG

(43) Veröffentlichungstag:
**16.02.2000 Patentblatt 2000/07**

(51) Int. Cl.⁷: **C07K 5/06**, C07K 5/02

(21) Anmeldenummer: **99100929.1**

(22) Anmeldetag: **28.08.1993**

(84) Benannte Vertragsstaaten:
**AT BE CH DE DK ES FR GB GR IE IT LI LU MC NL PT SE**

(30) Priorität: **03.09.1992 DE 4229447**
**22.12.1992 DE 4243496**

(62) Dokumentnummer(n) der früheren Anmeldung(en) nach Art. 76 EPÜ:
**93919208.4 / 0 610 487**

(71) Anmelder:
- **Boehringer Ingelheim Pharma KG**
  **55216 Ingelheim am Rhein (DE)**
  Benannte Vertragsstaaten:
  **BE CH DE DK ES FR GR IT LI LU MC NL PT SE AT**
- **Boehringer Ingelheim International GmbH**
  **55216 Ingelheim (DE)**
  Benannte Vertragsstaaten:
  **GB IE**

(72) Erfinder:
- **Esser, Franz**
  **55218 Ingelheim/Rhein (DE)**
- **Schnorrenberg, Gerd**
  **88400 Biberach/Riss (DE)**
- **Dollinger, Horst**
  **55218 Ingelheim/Rhein (DE)**
- **Jung, Birgit**
  **55270 Schwabenheim (DE)**
- **Buerger, Erich**
  **55411 Bingen/Rhein (DE)**

(74) Vertreter:
**Laudien, Dieter, Dr. et al**
**Boehringer Ingelheim GmbH**
**Abteilung Patente**
**55216 Ingelheim (DE)**

Bemerkungen:
Diese Anmeldung ist am 200199 als Teilanmeldung zu der unter INID-Kode 62 erwähnten Anmeldung eingereicht worden.

# (54) Peptide mit Tachykinin-antagonistischer Aktivität, Verfahren zu ihrer Herstellung und diese Verbindungen enthaltende pharmazeutische Zusammensetzungen

(57) Die Erfindung betrifft neue Aminosäurederivate der allgemeinen Formel I

$$R^1 - \overset{O}{\overset{\|}{C}} - A^1 - A^2 - NR^2R^3 \qquad (I)$$

und deren pharmazeutisch annehmbare Salze,
worin $R^1$, $A^1$, $A^2$, $R^2$ und $R^3$ die in der Beschreibung angegebenen Bedeutungen besitzen sowie deren Herstellung und Verwendung. Die neuen Verbindungen sind wertvolle Neurokinin (Tachykinin)- Antagonisten.

EP 0 979 827 A1

## Beschreibung

Die Erfindung betrifft neue Aminosäurederivate der allgemeinen Formel I,

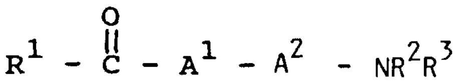

(I)

und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

In den europäischen Patentanmeldungen EP 394 989 und EP 443 132 werden Peptide mit Neurokinin antagonistischer Wirkung beschrieben. Die erfindungsgemäßen Verbindungen unterscheiden sich von diesen Peptiden wesentlich in den Gliedern $A^2$ und

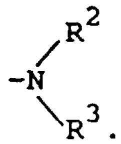

Die in dieser Beschreibung und den Ansprüchen für die Aminosäuren verwendeten Abkürzungen entsprechen dem üblichen Dreibuchstabencode wie er z.B. in Europ. J. Biochem., 138, 9 (1984) beschrieben ist. Die übrigen Abkürzungen werden nachfolgend erklärt:

| | |
|---|---|
| Boc | = t-Butoxycarbonyl |
| Bzl | = Benzyl |
| CDI | = Carbonyldiimidazol |
| Cha | = 3-Cyclohexylalanin |
| DCCI | = Dicyclohexylcarbodiimid |
| DCH | = Dicyclohexylharnstoff |
| HOBt | = 1-Hydroxybenztriazol |
| Hpa | = Homophenylalanin |
| Hyp | = (2S, 4R)-Hydroxyprolin |
| Pal | = 3-(1-Pyrrolyl)alanin |
| THF | = Tetrahydrofuran |
| TFA | = Trifluoressigsäure |
| Z | = Benzyloxycarbonyl |
| Me | = Methyl |
| Ac | = Acetyl |
| Et | = Ethyl |
| DMF | = Dimethylformamid |
| DPPA | = Diphenylphosphorylazid |
| PPA | = Polyphosphorsäure |
| RT | = Raumtemperatur |

Der Ausdruck Aminosäure umfaßt (falls im folgenden Text nicht ausdrücklich etwas anderes angegeben ist) natürliche und unnatürliche Aminosäuren, sowohl der D-als auch der L-Form, insbesondere α-Aminosäuren, sowie deren Isomere.

Wenn eine Aminosäure ohne Präfix angegeben ist (z.B. Orn) steht diese Angabe für die L-Form der Aminosäure. Die D-Form wird ausdrücklich angegeben.

Die Erfindung betrifft neue Aminosäurederivate der allgemeinen Formel I

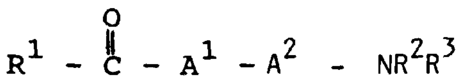 (I)

und deren pharmazeutisch annehmbare Salze, worin

$R^1$ Vinyl, Aryl, Heteroaryl, Aralkyl, Heteroaralkyl, Arylvinyl, Heteroarylvinyl, Aryloxyalkyl, Arylalkyloxy, ($C_3$-$C_8$) Cycloalkyl, ($C_3$-$C_8$) Cycloalkylalkyl, unsubstituiertes oder durch 1-3 Methylgruppen substituiertes Bicycloheptyl oder Bicycloheptylalkyl, Adamantyl, Adamantylalkyl, Dekalin, Dekalinalkyl, Tetralin, Tetralinalkyl, Diphenylalkyl, Di(arylalkyl)- aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl, Hydroxy, Nitro, Alkylcarbonyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin (Pro($NH_2$)) wie beispielsweise 3-Aminoprolin (Pro(3$NH_2$)) und 4-Aminoprolin (Pro(4$NH_2$)), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), Tpr($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$A^2$ eine lipophile α-Aminosäure ist, die eine Phenyl-, 1-, 2- oder 3-fach substituierte Phenyl-, Heteroaryl-, Cyclohexyl- oder Cyclopentylgruppe, eine Naphthylgruppe oder eine Mono- oder Di-$C_{1-3}$-alkylaminogruppe enthält, und diese Ringgruppe bzw. Aminogruppe durch eine 1- bis 8-gliedrige Kette vom Backbone der Aminosäure getrennt ist, (wobei die Substituenten der Phenylgruppe unabhängig voneinander Halogen, Trihalogenomethyl, Alkoxy, Alkyl, Cyano oder 1-Pyrrolidinyl sind und wobei in der 1- bis 8-gliedrigen Kette die Glieder der Kette -$CHR^4$, -C(O)-, -O-, -S- und/oder -$NR^4$-sein können, die so angeordnet sind, daß sich eine der folgenden 3 Kettenarten ergibt

$-(CHR^4)_{1-8}-$
$-(CHR^4)_{0-p}-G^1-(CHR^4)_{0-q}-$
$-(CHR^4)_{1-p}-G^2-(CHR^4)_{0-q}-$

worin $G^1$ -C(O)O- oder -C(O)-$NR^4$- ist, $G^2$ -O-, -S-, -$NR^4$-C(O)-O-, -$NR^4$-C(O)-, -$NR^4$-C(O)-$NR^4$- oder -O-C(O)-$NR^4$- ist und p und q ganze Zahlen von 1 bis 6 sind, die so gewählt werden, daß die Gesamtzahl der Kettenglieder 1 bis 8 ist,
und $R^4$ für Wasserstoff, Alkyl, Aryl oder Aralkyl steht, wobei

Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl oder Cyano sind; und die Alkylgruppe 1 bis 3 Kohlenstoffatome enthält;
(wobei, wenn eine Kette mehr als eine -$CHR^4$-Gruppe enthält, nur in einer dieser -$CHR^4$-Gruppen $R^4$ Alkyl, Aryl oder Aralkyl sein kann und
wobei die Kette nicht $CH_2$ ist, wenn $R^2$ und $R^3$ Alkyl oder Aralkyl sind und wenn

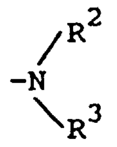

zusammen die Gruppe

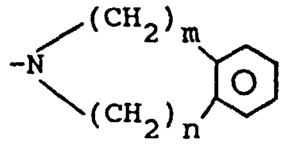

sind und m und n je 0, 1, 2 oder 3 sind, wobei deren Summe 2, 3, 4 oder 5 ergibt) oder $A^2$ Leu, Ile, Nle, Val, Met

oder eine der Gruppen

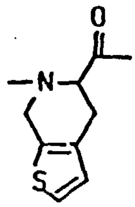

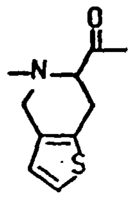

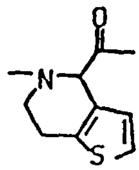

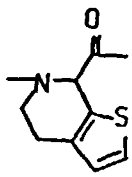

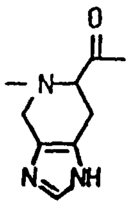

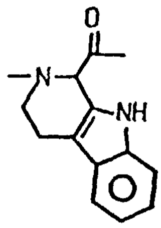

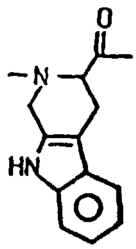

**und**

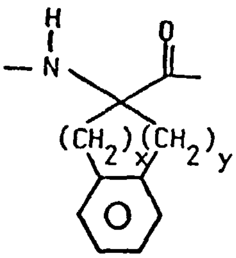

(worin x und y unabhängig voneinander 1 oder 2 sind) ist;

$R^2$ und $R^3$ unabhängig voneinander Alkyl, Arylalkyl, Heteroaryl oder Hydroxy bedeuten (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Alkylthio, Hydroxy, Nitro, Trifluormethoxy, Dialkylamino oder Cyano sind oder 2 benachbarte Positionen der Phenylgruppe durch -O-$(CH_2)_1$ oder $_2$-O-verbunden sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

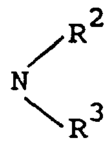

einen Ring der allgemeinen Formel

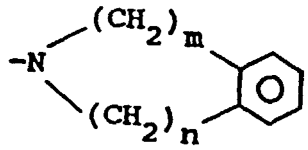 oder 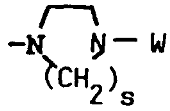

bedeuten, worin m und n je 0, 1, 2 oder 3 sind, wobei deren Summe 2, 3, 4 oder 5 ergibt, s 2 oder 3 ist,

W die Gruppe

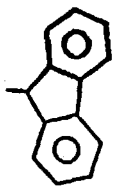, 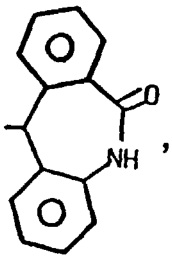, 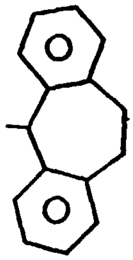

$(CH_2)_{0-2}$-Aryl, $CH(Aryl)_2$, Cyclopentyl, $(CH_2)_{0-2}$-Cyclohexyl, Pyridyl oder

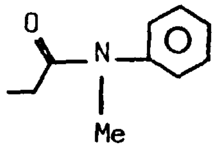

ist, (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Cyano, Hydroxy, Nitro oder Alkylthio sind oder 2 benachbarte Positionen der Phenylgruppe durch $-O-(CH_2)_{1-2}-O-$verbunden sind und Alkyl 1 bis 3 C-Atome enthält).

Verbindungen der allgemeinen Formel I können Säuregruppen besitzen, hauptsächlich Carboxylgruppen, oder phenolische Hydroxygruppen, und/oder basische Gruppen wie z.B. Guanidino- oder Aminofunktionen. Verbindungen der allgemeinen Formel I können deshalb entweder als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die Chiralitätszentren in den neuen Aminosäurederivate können jeweils R-, S- oder R,S-Konfiguration besitzen.

Der in der Definition von $A^2$ enthaltene Ausdruck "Heteroarylgruppe" steht für ein mono-, bi- oder tricyclisches aromatisches Ringsystem, das 1 oder 2 Heteroatome enthält, nämlich ein oder zwei Stickstoffatome oder ein Stickstoffatom und ein Schwefelatom. Die Gruppe kann gewünschtenfalls 1 oder 2 Substituenten ($C_{1-3}$Alkyl) oder eine Oxo-

gruppe oder eine Alkoxygruppe enthalten.

Beispiele geeigneter Heteroarylgruppen sind

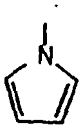 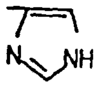 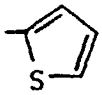 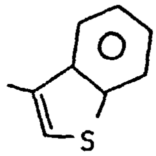 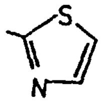

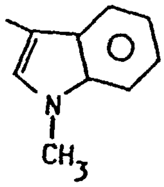 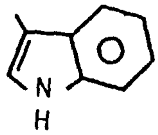 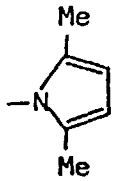 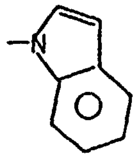

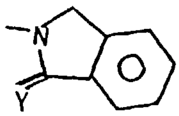 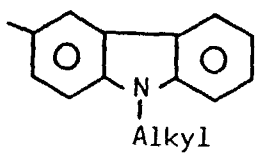 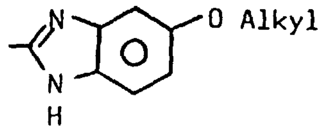

Y = $H_2$ oder O

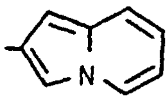 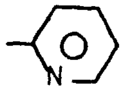

Es ist zu beachten, daß die oben angegebenen Heterarylgruppen auch in anderen Positionen als die angegebenen an die Kette gebunden sein können.

Die in $A^2$ enthaltene "1 bis 8 gliedrige Kette" besteht, wie oben ausgeführt worden ist, aus ein bis 8 Gliedern, worunter folgende Gruppen verstanden werden: $-CHR^4-$, -C(O)-, -0-, -S-, $-NR^4-$. Die Kette ist an das $\alpha$-Kohlenstoffatom der Aminosäure ($A^2$) gebunden.

$R^4$ steht (wie oben angegeben ist) für Wasserstoff, Alkyl, Aryl oder Aralkyl, vorzugsweise ist $R^4$ Wasserstoff, Methyl oder Phenyl.

Beispiele von geeigneten Ketten sind

$-(CH_2)_{1-4}-$
$-CH_2-O-CH_2-$, $-CH_2-O-$
$-CH_2-S-CH_2-$, $-CH_2-S-$
$-CH(CH_3)-O-CH_2-$, $-CH(CH_3)-O-$
$-(CH_2)_{1-2}-C(O)-O-CH_2-$, $-C(O)-NH-$
$-(CH_2)_4-NH-C(O)-O-CH_2-$
$-CH_2-C(O)-NH-$
$-CH_2-C(O)-NH-CH_2-$
$-CH_2-C(O)-N(CH_3)-CH_2-$
$-CH_2-C(O)-O-$
$-CH_2-NH-C(O)-CH_2-$
$-CH_2-NH-C(O)-O-$
$-CH_2-NH-C(O)-O-CH_2-$

$-CH_2-NH-C(O)-NH-$
$-(CH_2)_2-C(O)-NH-(CH_2)_2-$
$-(CH_2)_4-NH-C(O)-CH_2-$
$-(CH_2)_3-NH-C(O)-0-CH_2-$

Vorzugsweise enthält die Kette 1 bis 5, insbesondere 1 bis 4 Glieder.

Von den erfindungsgemäßen Verbindungen der Formel I sind solche bevorzugt, worin

$R^1$ Vinyl, Aryl, Heteroaryl, Aralkyl, Heteroaralkyl, Arylvinyl, Heteroarylvinyl, Aryloxyalkyl, Arylalkyloxy, Di(arylalkyl)-aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin ($Pro(NH_2)$) wie beispielsweise 3-Aminoprolin ($Pro(3NH_2)$) und 4-Aminoprolin ($Pro(4NH_2)$), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D-oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), $Tpr(O_2)$ oder $Met(O_2)$, bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$A^2$ eine lipophile Aminosäure ist, die eine Phenyl-, 1-, 2- oder 3-fach substituierte Phenyl-, Heteroaryl-, Cyclohexyl- oder Cyclopentylgruppe oder eine Mono- oder Di-$C_{1-3}$ -alkylaminogruppe enthält, und diese Ringgruppe bzw. Aminogruppe durch eine 1- bis 8-gliedrige Kette vom Backbone der Aminosäure getrennt ist, (wobei die Substituenten der Phenylgruppe unabhängig voneinander Halogen, Trihalogenomethyl, Alkoxy, Alkyl, Cyano oder 1-Pyrrolidinyl sind und die Kette wie in Anspruch 1 definiert ist) oder $A^2$ Leu, Ile, Nle, Val, Met
oder eine der Gruppen

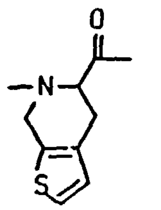 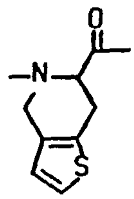 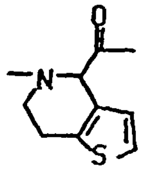 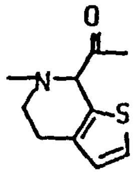

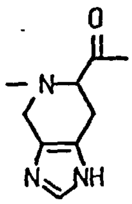 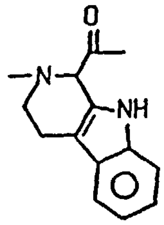 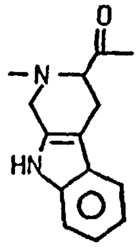

und 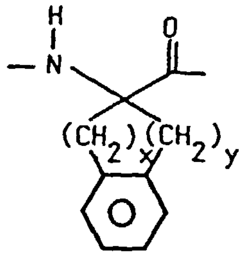

(worin x und y unabhängig voneinander 1 oder 2 sind) ist;

$R^2$ und $R^3$ unabhängig voneinander Alkyl, Arylalkyl, Heteroaryl oder Hydroxy bedeuten (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

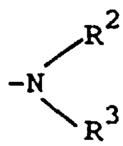

einen Ring der allgemeinen Formel

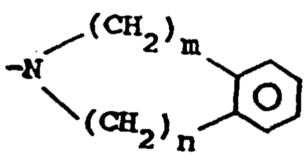 oder 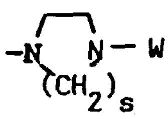

bedeuten, worin m, n und s wie in Anspruch 1 definiert sind, und

W die Gruppe

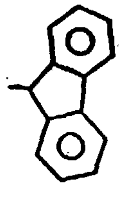 , 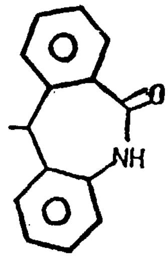

-$(CH_2)_{0-2}$-Aryl, CH$(Aryl)_2$, Cyclopentyl oder $(CH_2)_{0-2}$-Cyclohexyl ist, (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl oder Cyano sind).

Von den erfindungsgemäßen Verbindungen der Formel I

$$R^1\text{-C(O)-}A^1\text{-}A^2\text{-}NR^2R^3 \qquad \text{I}$$

sind solche bevorzugt worin

$R^1$ Aryl, Heteroaryl, Aralkyl, Heteroaralkyl, Aryloxyalkyl, Arylalkyloxy, Di(arylalkyl)-aminoalkyl (worin Aryl für Phenyl oder 1- oder 2-fach substituiertes Phenyl steht; die Substituenten der Phenylgruppe unabhängig voneinander Halogen oder Alkoxy sind; Heteroaryl für Indolyl, in Stellung 1 durch Alkyl oder Benzyl substituiertes Indolyl oder Pyridyl steht; und die Alkyl- bzw. Alkoxygruppe 1 bis 3 Kohlenstoffatome enthält) bedeutet; insbesondere

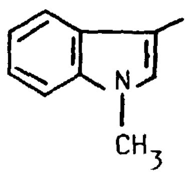

und/oder

$A^1$ eine Aminosäure ist, die eine oder 2 polare funktionelle Gruppe(n) in der Seitenkette trägt, wie OH, COOH, $NH_2$, Guanidin, $CONH_2$, SH; insbesondere worin die funktionelle Gruppe in der Seitenkette von $A^1$ OH ist und/oder worin $A^1$ Pro, 4-Hydroxyprolin, 3-Hydroxyprolin, Ser, Thr, Trp(For) oder Tyr ist; vorzugsweise 4-Hydroxyprolin mit 2-S-Konfiguration ist, insbesondere

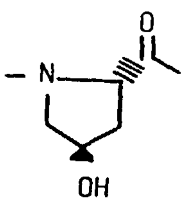

und /oder worin $A^2$ für eine acyclische oder cyclische Aminosäure wie (O-Benzyl)Ser, (O-subst. Benzyl)Ser, (O-Benzyl)Thr, Cyclohexylalanin, Homophenylalanin, 3-(1-Pyrrolyl)- alanin, 3-(2,5-Dimethyl-1-pyrrolyl)alanin, 3-(1-Indolyl)alanin, 2-Amino-4-(1-Pyrrolyl)- buttersäure, 2-Amino-5-(1-Pyrrolyl)valeriansäure, 2-Amino-6-(1-Pyrrolyl)capronsäure, Leu, Lys(Z), 3-(2-Thienyl)alanin, 3-(3-Benzothienyl)alanin, 3-(1-Isoindolinoyl)alanin, (O-Benzyl)Asp, (O-Benzyl)Glu, Trp, (N-Me)Trp, His, 3-(2-Thiazolyl)-alanin, oder 3-Dimethylamino-alanin, -(O-Methyl)Tyr, 2-Naphthylalanin,

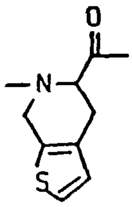 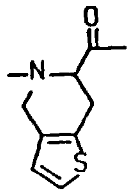 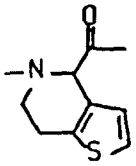 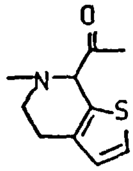

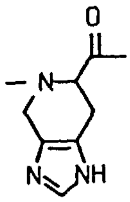 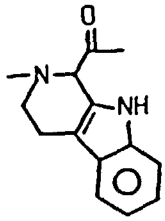 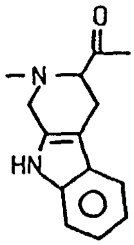

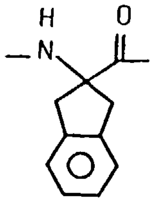 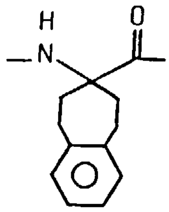 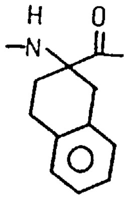 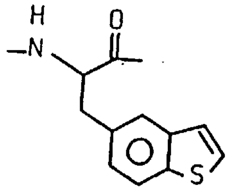

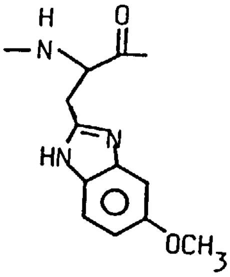 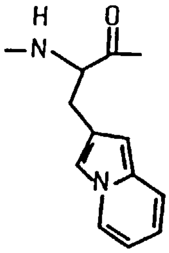 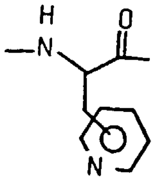

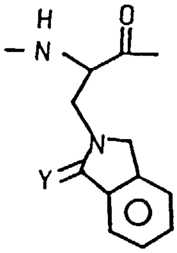 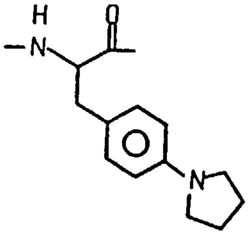 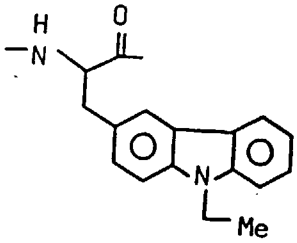

Y = $H_2$ oder O

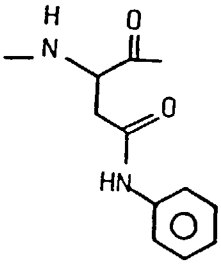 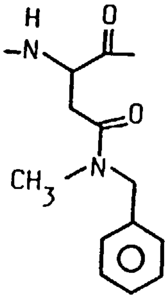 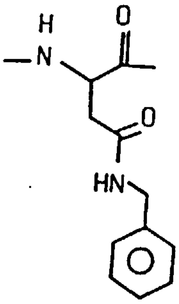

ist,
wobei die in den Aminosäuren enthaltenen Phenylgruppen 1-, 2- oder 3-fach substituiert sein können und die Substituenten unabhängig voneinander Halogen, Trihalogenomethyl, Alkoxy, Alkyl oder Cyano sind, die Alkyl- bzw. Alkoxygruppe 1 bis 3 Kohlenstoffatome enthält;
und wobei die angegebenen Aminosäuren vorzugsweise in S-Konfiguration vorliegen;

besonders hervorzuheben sind Verbindungen worin

$A^2$

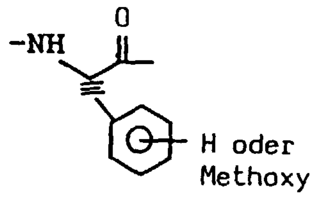

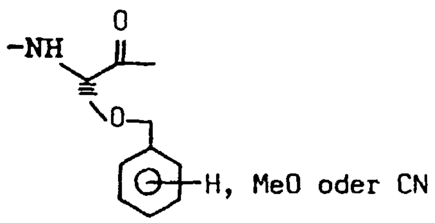

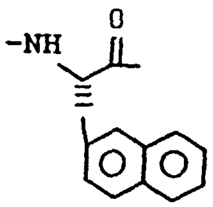

oder

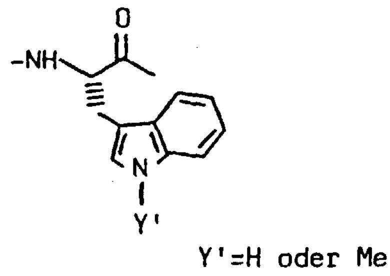

ist;
und/oder worin $R^2$ und $R^3$ unabhängig voneinander Methyl, Benzyl, Phenethyl (wobei die darin enthaltenen Phenylgruppen durch eine oder zwei Methoxygruppen substituiert sind) oder Pyridylmethyl ist; vorzugsweise Verbindung worin $R^2$ Methyl ist und $R^3$ Benzyl oder Alkoxybenzyl ist, insbesondere worin $R^3$ Alkoxybenzyl, vorzugsweise 2-Methoxybenzyl ist; oder worin die Gruppe

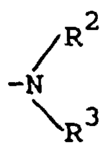

einen Ring

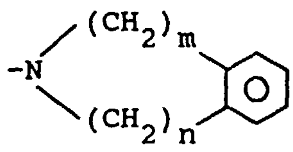

bedeutet, worin m 1 ist und n 1 oder 2;
oder worin die Gruppe

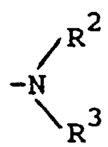

einen Ring

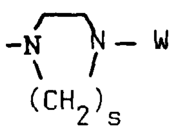

bedeutet, worin s 2 oder 3 ist (vorzugsweise 2) und W wie oben definiert ist;
vorzugsweise worin W

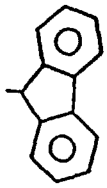

,

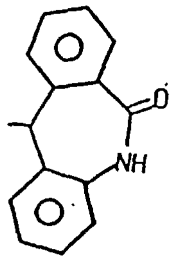

Cyclohexyl, Phenyl oder $CH(Phenyl)_2$ ist, worin die Phenylgruppen substituiert sind;
worin wenn W Phenyl ist, dieses vorzugsweise monosubstituiert ist durch Halogen, Alkoxy, Alkyl, Cyano, Hydroxy, Nitro oder Alkylthio, insbesondere durch Methoxy, Chlor, Methyl, Ethyl, Cyano, Hydroxy, Nitro oder Methylthio, vorzugsweise durch Methoxy, Chlor, Methyl, Cyano oder Methylthio, wobei der Substituent der Phenylgruppe vorzugsweise in Position 2 ist und
wenn W die Gruppe $-CH(Phenyl)_2$ ist, die Phenylgruppe je durch ein Halogen substituiert sind, vorzugsweise durch Fluor, wobei in der $-CH(Phenyl)_2$-gruppe die beiden Phenylgruppen vorzugsweise identisch substituiert sind, vorzugsweise in p-Stellung.

Die angegebenen Aminosäuren liegen vorzugsweise in S-Konfiguration vor.
Untersuchungsergebnisse für erfindungsgemäße Verbindungen:
Die Rezeptoraffinität zum $NK_1$-Rezeptor (Substanz P-Rezeptor) wurde an humanen Lymphoblastoma-Zellen (IM-9) mit klonierten $NK_1$-Rezeptoren bestimmt, wobei die Verdrängung von $^{125}$J-markierter Substanz P gemessen wird. Die so erhaltenen $IC_{50}$-Werte betragen:

| | |
|---|---|
| Verb. A : | 60 nM |
| Verb. B : | 21 nM |
| Verb. C : | 90 nM |
| Verb. D : | 45 nM |

Verbindung A

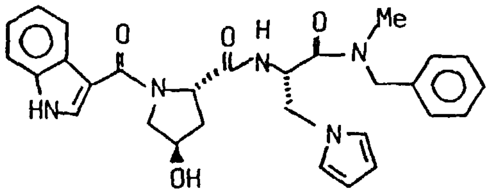

Verbindung B

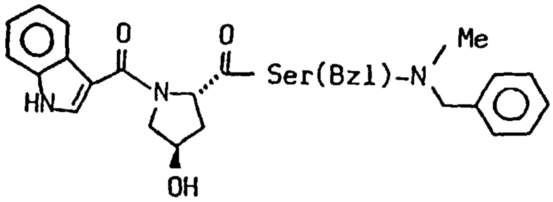

Verbindung C

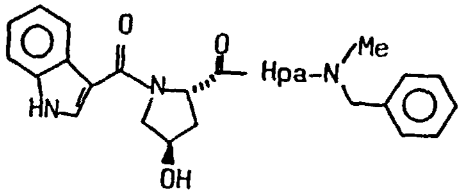

Verbindung D

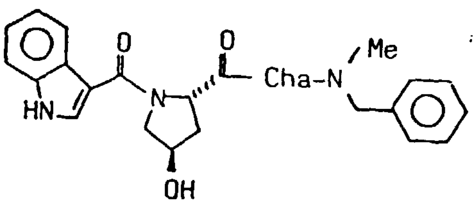

Die erfindungsgemäßen Verbindungen sind wertvolle Neurokinin (Tachykinin)-Antagonisten, die insbesondere Substanz P-Antagonismus, aber auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten wie Atemwegserkrankungen, z.B. Athsma, Bronchititis, Rhinitis, Husten oder Auswurf sowie von entzündlichen Augenkrankheiten wie beispielsweise Konjunktivitis, von entzündlichen Hautkrankheiten wie beispielsweise Dermatitis und Urticaria, von

anderen Entzündungskrankheiten wie Polyarthritis oder Osteoarthritis sowie von Schmerzzuständen und Erbrechen.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch Iontophorese oder literaturbekannte Enhancer gefördert, und oral erfolgen.

Zur parenteralen Applikation werden die Verbindungen der Formel I oder deren physiologisch verträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die erfindungsgemäßen Verbindungen können nach allgemein bekannten Methoden der Amino- und Peptidchemie hergestellt werden, indem man schrittweise die jeweiligen Aminosäuren, beziehungsweise Peptidderivatteilsequenzen, Carbonsäuren und Amine kondensiert und die so erhaltene Verbindung in freier Form oder in Form des gewünschten Salzes isoliert.

Die erfindungsgemäßen Dipeptidderivate der Formel I

$$R^1\text{-C(O)-}A^1\text{-}A^2\text{-NR}^2R^3 \qquad \text{I}$$

können aus den Teilen $R^1$-COOH, H-$A^1$-OH, H-$A^2$-OH und HN($R^3$)$R^2$ aufgebaut werden, wobei die Sequenz der Kupplungen von rechts nach links, von links nach rechts oder durch Kupplung der Einheiten

$$R^1\text{-CO-}A^1\text{-OH und H-}A^2\text{-N(}R^3\text{)}R^2$$

(Fragmentkupplungen) erfolgen kann.

Die erfindungsgemäßen Verbindungen können nach allgemein bekannten Methoden der Peptidchemie, wie z.B. in "Houben-Weyl, Methoden der organischen Chemie, Bd. 15/2", beschrieben oder nach der Festphasenpeptidsynthese (z.B. R.C. Sheppard, Int. J. Pept. Prot. Res., 21, 118 [1983]) oder gleichwertigen bekannten Methoden hergestellt werden. Dabei werden die jeweiligen Aminosäuren oder Aminosäureteilsequenzen schrittweise kondensiert und die so erhaltenen Peptide werden in freier Form oder in Form der gewünschten Salze isoliert. Als Aminoschutzgruppen werden die in "Houben-Weyl, Methoden der organischen Chemie, Bd. 15/1" beschriebenen verwendet, wobei in konventionellen Synthesen die Benzyloxycarbonylgruppe (Z) und in Festphasensynthesen die Fluorenylmethoxycarbonylgruppe (Fmoc) bevorzugt wird. Die Seitenkette des Arginins wurde im Fall der konventionellen Synthese durch Protonierung geschützt, im Fall der Festphasensynthese wurde die Mtr-Gruppe verwendet. In der Festphasenpeptidsynthese wurden z.B. auch folgende seitenkettengeschützte Aminosäuren eingesetzt: Lys(Boc), His(Bum), Ser(tBu) und Asp(tBu). Die speziellen Synthesebedingungen sind den nachfolgenden Beispielen zu entnehmen.

Zur Synthese der Verbindungen der allgemeinen Formel I nach der Festphasensynthese werden zunächst die Dipeptidcarbonsäuren synthetisiert, die in Lösung zu den Dipeptidamiden umgesetzt werden. Als Ankergruppen sind folgende geeignet

1. Benzylester (G. Barang, R.B. Merrifield, Peptides 2, 1 (1980) Eds. E. Gross, J. Meienhofer, Academic Press, New York)
2. PAM-Anker (R.B. Merrifield, J. Am. Chem. Soc. 85, 2149 (1966))
3. Wang-Anker (S.-S. Wang, J. Am. Chem. Soc. 95, 1328 (1973))
4. SASRIN-Anker (M. Mergler, R. Tanner, J. Gostuli, P. Grogg, Tetrah. Lett. 29, 4005 (1988)).

## Pharmazeutische Zubereitungen:

### Injektionslösung

| | | | |
|---|---|---|---|
| 200 mg | Wirksubstanz * | | |
| 1,2 mg | Monokaliumdihydrogenphosphat = $KH_2PO_4$ | (Puffer) | |
| 0,2 mg | Dinatriumhydrogenphosphat = $NaH_2PO_4 \cdot 2H_2O$ | (Puffer) | |
| 94 mg | Natriumchlorid | (Isotonans) | |
| oder | | | |
| 520 mg | Glucose | (Isotonans) | |
| 4 mg | Albumin | (Proteasenschutz) | |
| q.s. | Natronlauge | ad pH 6 | |
| q.s. | Salzsäure | ad pH 6 | |
| ad 10 ml | Wasser für Injektionszwecke | | |

### Injektionslösung

| | | |
|---|---|---|
| 200 mg | Wirksubstanz* | |
| 94 mg | Natriumchlorid | |
| oder | | |
| 520 mg | Glucose | |
| 4 mg | Albumin | |
| q.s. | Natronlauge | ad pH 9 |
| q.s. | Salzsäure | ad pH 9 |
| ad 10 ml | Wasser für Injektionszwecke | |

### Lyophilisat

| | |
|---|---|
| 200 mg | Wirksubstanz* |
| 520 mg | Mannit (Isotonans/Gerüstbildner) |
| 4 mg | Albumin |

Lösungsmittel 1 für Lyophilisat

| | | |
|---|---|---|
| 10 | ml | Wasser für Injektionszwecke |

Lösungsmittel 2 für Lyophilisat

| | | |
|---|---|---|
| 20 | mg | Polysorbat® 80 = Tween® 80 (oberflächenaktiver Stoff) |
| 10 | ml | Wasser für Injektionszwecke |

* Wirksubstanz: erfindungsgemäße Verbindungen, z.B. die des Beispiels 1

Dosis für Mensch von 67 kg: 1 bis 500 mg

aufgenommen und über eine kurze Kieselgelsäule chromatrographiert. Aus dem Eluat erhält man nach Einengen 12,5 g Z-Pal-OMe als farbloses Öl.

$[\alpha]_D^{20}$ (MeOH) = -26,8°.

Herstellung von Z-Pal-OH:
5,5 g Z-Pal-OMe (18,2 mMol) werden in 100 ml Acetonitril gelöst, mit 20 ml 1N NaOH versetzt und 3 Stunden bei Raumtemperatur gerührt. Die Mischung wird durch Zugabe von 20 ml 1 N NaOH neutralisiert, das Lösungsmittel zum größten Teil am Rotationsverdampfer abgezogen und der feste Rückstand mit eiskaltem Wasser versetzt. Nach Absaugen wäscht man mit wenig Wasser und trocknet den Rückstand im Exsikkator wobei man Z-Pal-OH in Form weißer Kristalle erhält.

Fp.: 82°C; $[\alpha]_D^{20}$ (MeOH) = -9,4°.

Herstellung von Z-Pal-N(Me)Bzl:
2,5 g Z-Pal-OH (8,7 mMol) werden in 50 ml THF gelöst, mit 1,55 g CDI (9,5 mMol) versetzt, 45 Minuten bei Raumtemperatur gerührt, dann mit 1,12 ml N-Methylbenzylamin (8,7 mMol) versetzt und weitere 64 Stunden bei Raumtemperatur gerührt. Die Reaktionsmischung wird am Rotationsverdampfer eingeengt, der Rückstand in Essigester aufgenommen, 1 x mit kaltem Wasser und 2 x mit 10 %iger NaCl-Lösung ausgeschüttelt. Die organische Phase wird filtriert, eingeengt und mittels Ether als Fließmittel über eine Kieselgelsäule chromatrographiert. Aus dem Eluat wird Z-Pal- N(Me)Bzl als farbloses Öl gewonnen.

$[\alpha]_D^{20}$ (MeOH) = -0,3°.

Herstellung von H-Pal-N(Me)Bzl:
2,68 g Z-Pal-N(Me)Bzl (6,85 mMol) werden in 30 ml MeOH gelöst und nach Zusatz von 0,3 g Pd-Kohle über 5 Stunden bei Raumtemperatur und 5 Bar hydriert. Danach wird filtriert, das Filtrat am Rotationsverdampfer eingeengt, der Rückstand in Ether gelöst, filtriert und das Filtrat erneut eingeengt. Man erhält H-Pal-N(Me)Bzl als hellgrünliches Öl.

$[\alpha]_D^{20}$ (MeOH) = +38,8°.

Herstellung von H-Hyp-Pal-N(Me)Bzl:
1,52 g H-Pal-N(Me)Bzl (5,9 mMol), 1,37 g Boc-(2S,4R)-Hydroxy-prolin (5,9 mMol) und 0,91 g HOBt. $H_20$ (5,9 mMol) werden in 120 ml THF gelöst, auf 3°C abgekühlt und mit 1,83 g DCCI (8,9 mMol) versetzt. Man rührt 2 Stunden bei 3°C

und weitere 13 Stunden bei Raumtemperatur, dann wird vom angefallenen DCH abfiltriert, das Filtrat eingeengt und der Rückstand in Acetonitril aufgenommen. Man filtriert erneut, engt das Filtrat ein, nimmt in Essigester auf und wäscht die organische Phase 2 x mit gesättigter $NaHCO_3$-Lösung und 3 x mit 10 %iger NaCl-Lösung. Nach Trocknen mittels $MgSO_4$ und Chromatographie über Kieselgel mit Essigester als Fließmittel erhält man Boc-Hyp-Pal-N(Me)Bzl als weißen, festen Schaum.

Das erhaltene Boc-Hyp-Pal-N(Me)Bzl wird in 40 ml $CH_2Cl_2$ gelöst und unter Eiskühlung mit 20 ml TFA versetzt. Man rührt 15 Minuten bei Raumtemperatur, engt am Rotationsverdampfer ein, nimmt den Rückstand in Essigester auf und extrahiert 2 x mit gesättigter $NaHCO_3$-Lösung. Die wässerige Phase wird nacheinander mit Essigester und $CH_2Cl_2$ ausgeschüttelt. Alle organischen Phasen werden vereint, mit $MgSO_4$ getrocknet, eingeengt und der Rückstand über Kieselgel mittels Acetonitril/Wasser/MeOH (4:1:1) chromatrographiert. Es wird 0,39 g H-Hyp-Pal-N(Me)Bzl als hochviskoses, farbloses Öl erhalten.

$[\alpha]_D^{20}$ (MeOH) = -38,1°.

Herstellung von 3-Indolylcarbonyl-Hyp-Pal-N(Me)Bzl:
0,32 g H-Hyp-Pal-N(Me)Bzl (0,9 mMol) werden in 30 ml $CH_2Cl_2$ gelöst, bei Raumtemperatur mit 0,52 ml Bis(trimethylsilyl)acetamid (2,1 mMol) versetzt, 40 Minuten bei Raumtemperatur gerührt, bei 0°C mit 0,19 g Indol-3-carbonsäurechlorid (1 mMol) versetzt, weitere 2 Stunden bei 0°C und schließlich 1 Stunde bei Raumtemperatur gerührt. Das Reaktionsgemisch wird am Rotationsverdampfer eingeengt, der Rückstand in 25 ml THF und 7 ml 1N NaOH aufgenommen, und nach 1-stündigem Rühren durch Zugabe von 7 ml 1N HCl neutralisiert. Das THF wird am Rotationsverdampfer abdestilliert, die resultierende wässerige Phase 2 x mit Essigester extrahiert, die vereinigten Essigesterphasen filtriert und das Filtrat eingeengt. Nach Chromatographie über Kieselgel mit Essigester/MeOH (9:1) als Elutionsmittel erhält man 3-Indolylcarbonyl-Hyp-Pal-N(Me)Bzl als weißen Feststoff.

Fp.: 112-116°C.
$[\alpha]_D^{20}$ (MeOH) = -124,4°.

In den folgenden Tabellen werden weitere Verbindungen zusammengefaßt, die in analoger Weise hergestellt werden können.

Tabelle 1

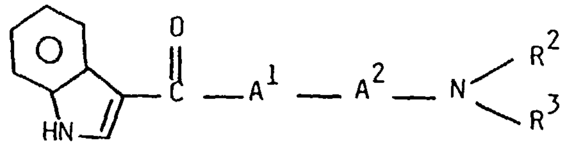

| Nr. | $A^1$ | $A^2$ | 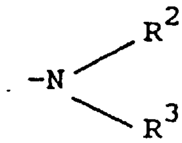 |
|---|---|---|---|
| 1 | 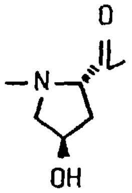 | 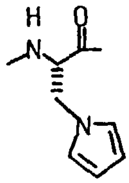 | 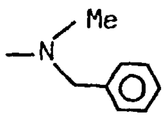 |
| 2 | 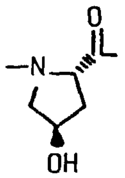 | Ser(Bzl) | 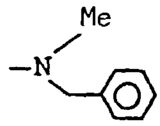 |
| 3 | 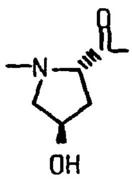 | Hpa | 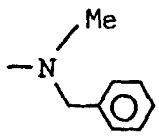 |
| 4 | 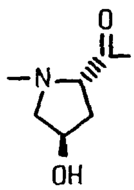 | Cha | 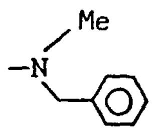 |

| Nr. | $A^1$ | $A^2$ | 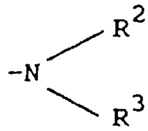 |
|---|---|---|---|
| 5 | Hyp | 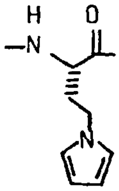 | —N(Me)Bzl |
| 6 | Hyp | 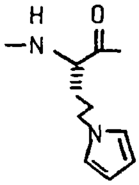 | —N(Me)Bzl |
| 7 | Hyp | 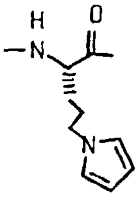 | 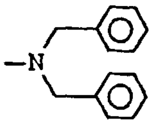 |
| 8 | Hyp | 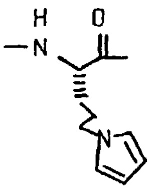 | 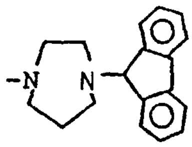 |
| 9 | Hyp | 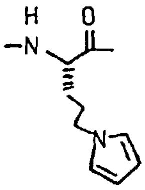 | 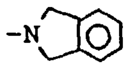 |
| 10 | Hyp | 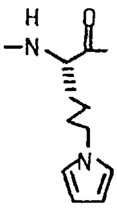 | —N(Me)Bzl |

| Nr. | $A^1$ | $A^2$ | 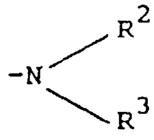 |
|---|---|---|---|
| 12 | Hyp | 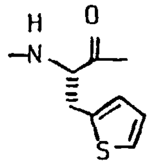 | —N(Me)Bzl |
| 13 | Hyp | 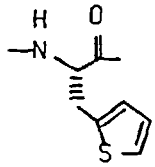 | 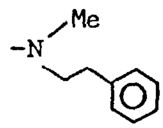 |
| 14 | Hyp | 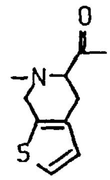 | —N(Me)Bzl |
| 15 | Thr | 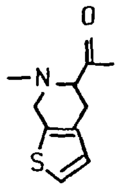 | —N(Me)Bzl |
| 16 | Hyp | 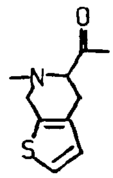 | 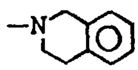 |

| Nr. | $A^1$ | $A^2$ | 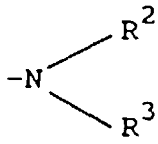 |
|---|---|---|---|
| 17 | Hyp | 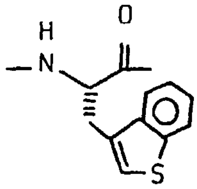 | -N(Me)Bzl |
| 20 | Hyp | 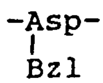 | -N(Me)Bzl |
| 22 | Hyp | 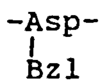 | 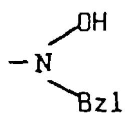 |

| Nr. | $A^1$ | $A^2$ | 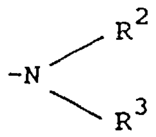 |
|---|---|---|---|
| 23 | Hyp | -Glu-<br>Bzl | −N(Me)Bzl |
| 24 | Hyp | -Glu-<br>Bzl | 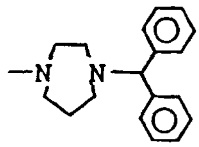 |
| 25 | Hyp | 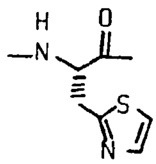 | −N(Me)Bzl |
| 26 | Hyp | 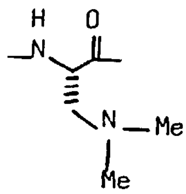 | −N(Me)Bzl |
| 27 | Hyp | 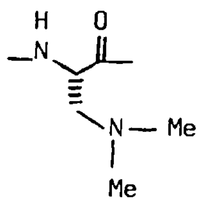 | $-N(Bzl)_2$ |
| 28 | Hyp | 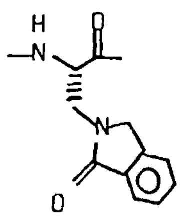 | −N(Me)Bzl |

| Nr. | $A^1$ | $A^2$ | 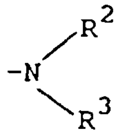 |
|---|---|---|---|
| 29 | Hyp | 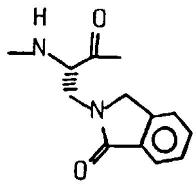 | 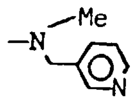 |
| 30 | Pro | Ser<br>Bzl | –N(Me)Bzl |
| 31 | Hyp | Ser<br>Bzl | 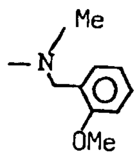 |
| 32 | Hyp | Ser<br>Bzl | 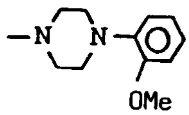 |
| 33 | Hyp | Ser<br>Bzl | 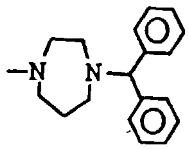 |
| 34 | Hyp | D-Ser<br>Bzl | –N(Me)Bzl |

| Nr. | $A^1$ | $A^2$ | 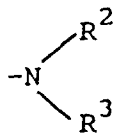 |
|---|---|---|---|
| 35 | Hyp | Ser 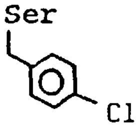 | -N(Me)Bzl |
| 36 | Hyp | Ser 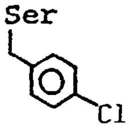 | 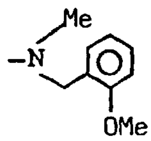 |
| 37 | Hyp | Ser 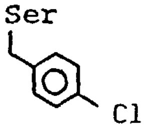 | 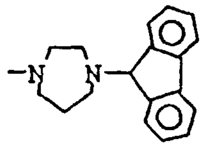 |
| 38 | Hyp | Leu | -N(Me)Bzl |
| 39 | Hyp | Thr<br>Bzl | -N(Me)Bzl |
| 40 | Hyp | Thr<br>Bzl | 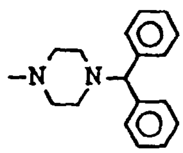 |

| Nr. | $A^1$ | $A^2$ | 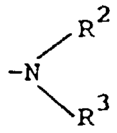 |
|---|---|---|---|
| 41 | Hyp | Lys<br>\|<br>Z | 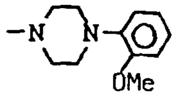 |
| 42 | Hyp | 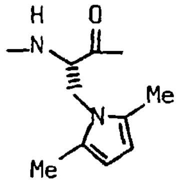 | -N(Me)Bzl |
| 43 | Hyp | 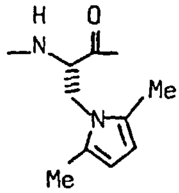 | 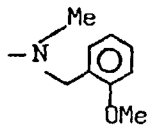 |
| 44 | Hyp | 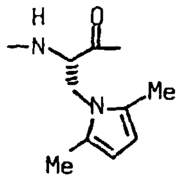 | 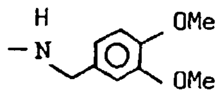 |
| 45 | Hyp | 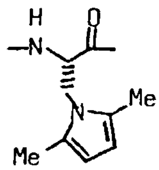 | 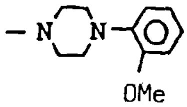 |
| 46 | Hyp | 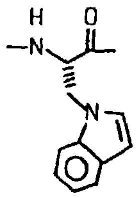 | -N(Me)Bzl |

| Nr. | $A^1$ | $A^2$ | 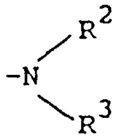 |
|---|---|---|---|
| 47 | Hyp | 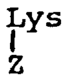 | 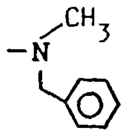 |
| 48 | Hyp | 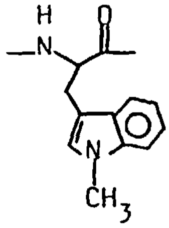 | −N(Me)Bzl |

Tabelle 2

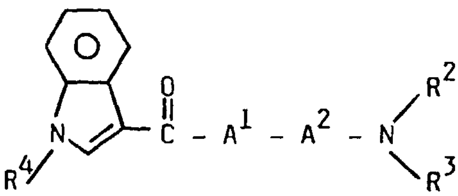

| Nr. | $R^4$ | $A^1$ | $A^2$ | 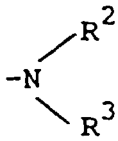 |
|---|---|---|---|---|
| 49 | $CH_3$ | Hyp | 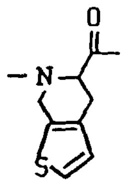 | −N(Me)Bzl |

| Nr. | $R^4$ | $A^1$ | $A^2$ | 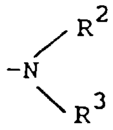 |
|---|---|---|---|---|
| 50 | Bzl | Hyp | 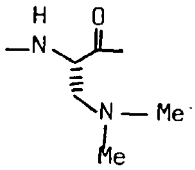 | −N(Me)Bzl |
| 51 | Me | Hyp | Trp | −N(Me)Bzl |
| 52 | Me | Hyp | His | −N(Me)Bzl |
| 53 | $CH_3$ | Hyp | Lys(Z) | −N(Me)Bzl |
| 54 | $CH_3$ | Hyp | 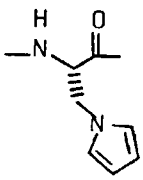 | −N(Me)Bzl |
| 55 | $CH_3$ | Hyp | Ser(Bzl) | −N(Me)Bzl |
| 55a | $CH_3$ | Hyp | Ser(p-CN-Bzl) | 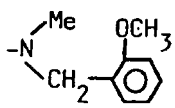 |

| Nr. | $R^4$ | $A^1$ | $A^2$ | 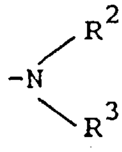 |
|---|---|---|---|---|
| 56 | $CH_3$ | Hyp | Hpa | −N(Me)Bzl |
| 57 | $CH_3$ | Hyp | Cha | −N(Me)Bzl |

Tabelle 3

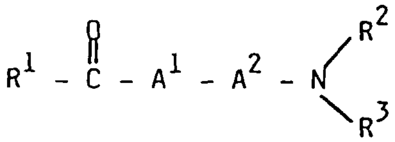

| Nr. | $R^1$ | $A^1$ | $A^2$ | 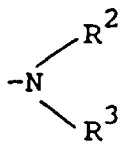 |
|---|---|---|---|---|
| 58 | 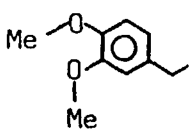 | Hyp | Hpa | −N(Me)Bzl |

| Nr. | $R^1$ | $A^1$ | $A^2$ | 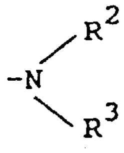 |
|---|---|---|---|---|
| 59 | 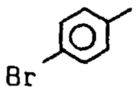 | Hyp | 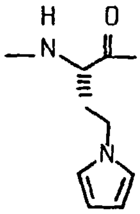 | —N(Me)Bzl |
| 60 | 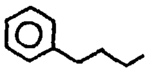 | Hyp | 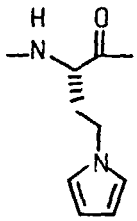 | —N(Me)Bzl |
| 61 | 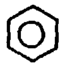 | Hyp | 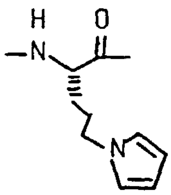 | —N(Me)Bzl |
| 62 | 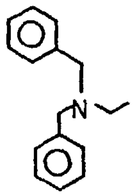 | Hyp | 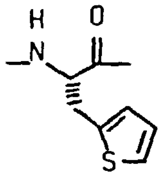 | —(N(Me)Bzl |

| Nr. | $R^1$ | $A^1$ | $A^2$ | 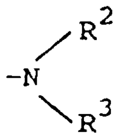 |
|---|---|---|---|---|
| 65 | 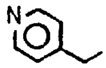 | Hyp | Glu<br>Bzl | −N(Me)Bzl |
| 66 | 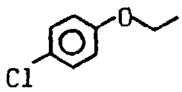 | Hyp | 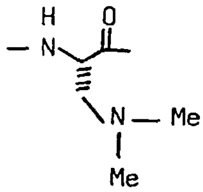 | −N(Me)Bzl |
| 67 | 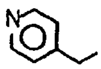 | Hyp | 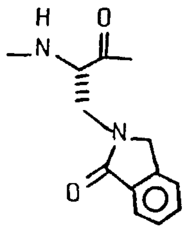 | −N(Me)Bzl |
| 68 | 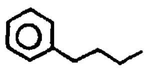 | Hyp | Ser<br>Bzl | −N(Me)Bzl |
| 69 | 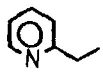 | Hyp | Ser<br>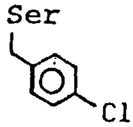 | −N(Me)Bzl |
| 70 | 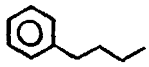 | Hyp | Lys<br>Z | 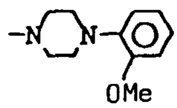 |

| Nr. | $R^1$ | $A^1$ | $A^2$ | 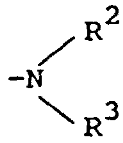 |
|---|---|---|---|---|
| 71 | 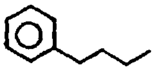 | Hyp | Lys<br>Z | -N(Me)Bzl |
| 72 | 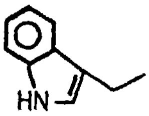 | Hyp | 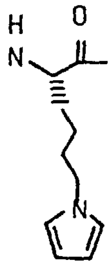 | -N(Me)Bzl |

Tabelle 4

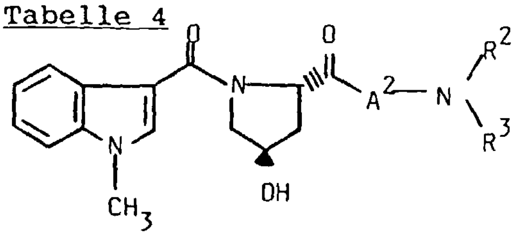

| Nr. | $A^2$ | 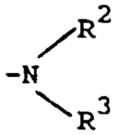 |
|---|---|---|
| 73 | 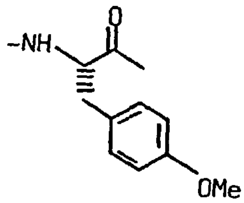 | 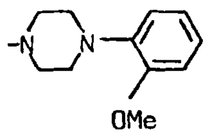 |
| 74 | 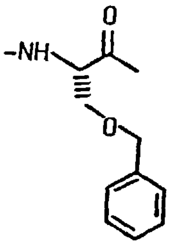 | 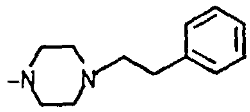 |
| 75 | 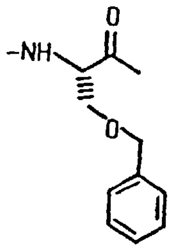 | 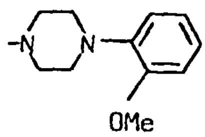 |
| 76 | 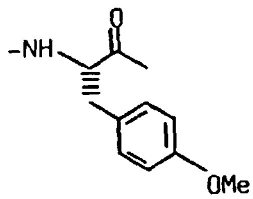 | 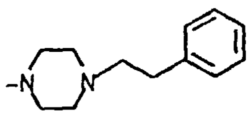 |

| Nr. | $A^2$ | 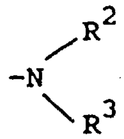 |
|---|---|---|
| 77 | 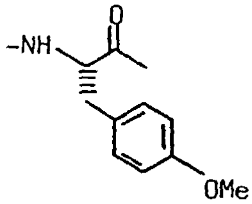 | 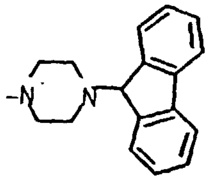 |
| 78 | 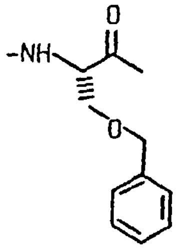 | 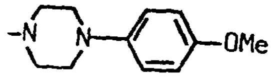 |
| 79 | 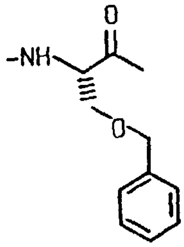 | 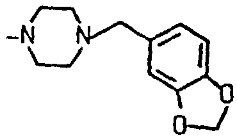 |
| 80 | 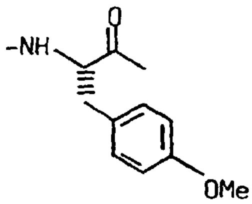 | 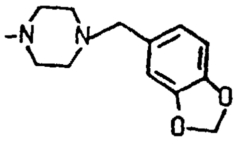 |
| 81 | 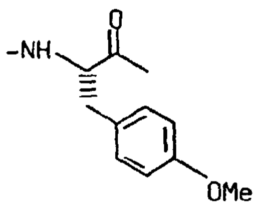 | 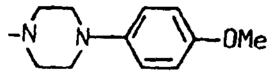 |
| 82 | 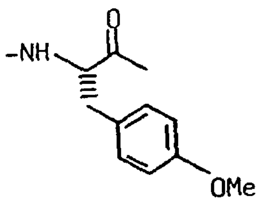 | 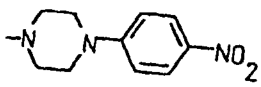 |

| Nr. | $A^2$ | 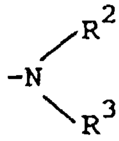 |
|---|---|---|
| 83 | 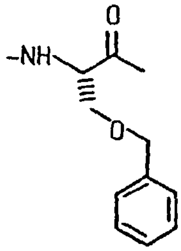 | 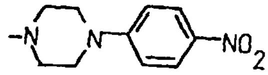 |
| 84 | 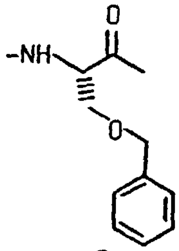 | 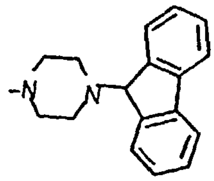 |
| 85 | 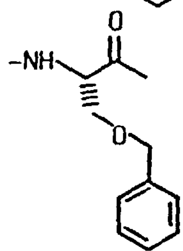 | 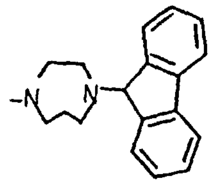 |
| 86 | 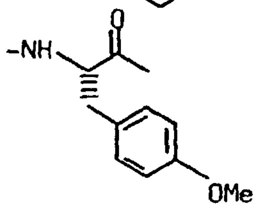 | 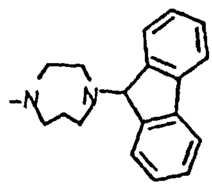 |
| 87 | 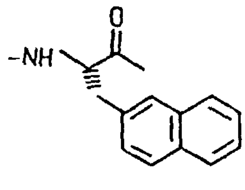 | 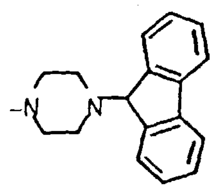 |
| 88 | 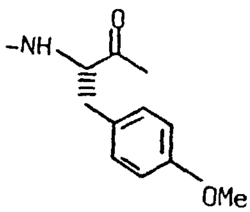 | 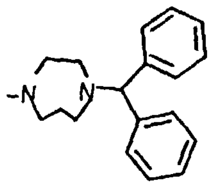 |

| Nr. | $A^2$ | 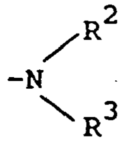 |
|---|---|---|
| 89 | 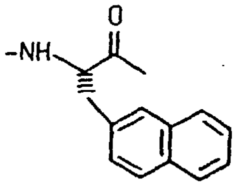 | 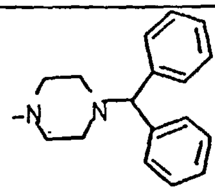 |
| 90 | 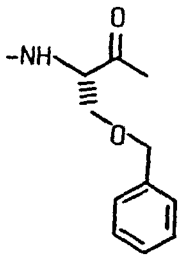 | 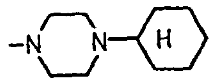 |
| 91 | 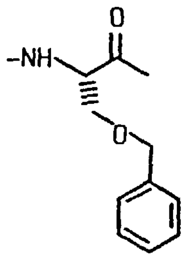 | 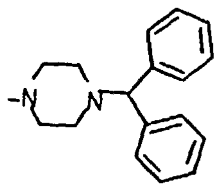 |
| 92 | 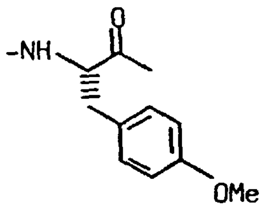 | 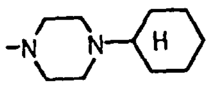 |
| 93 | 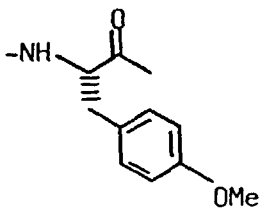 | 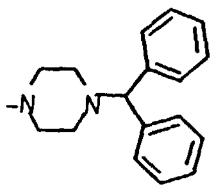 |
| 94 | 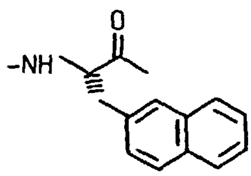 | 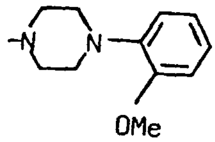 |

| Nr. | $A^2$ | 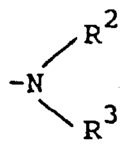 |
| --- | --- | --- |
| 95 | 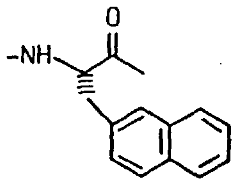 | 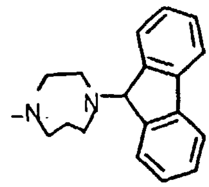 |
| 96 | 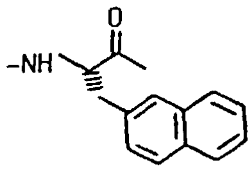 | 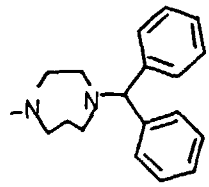 |
| 97 | 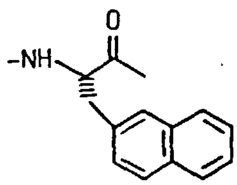 | 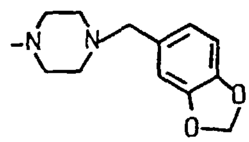 |
| 98 | 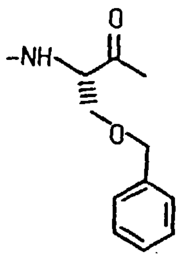 | 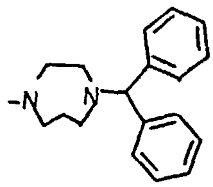 |
| 99 | 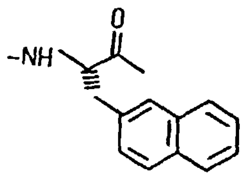 | 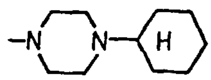 |
| 100 | 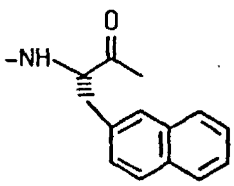 | 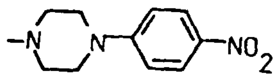 |

| Nr. | $A^2$ | 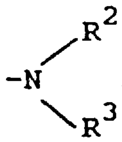 |
|---|---|---|
| 101 | 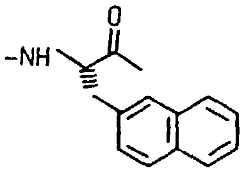 | 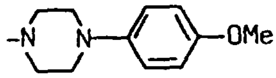 |
| 102 | 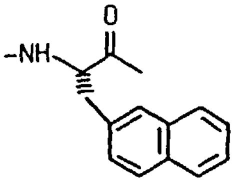 | 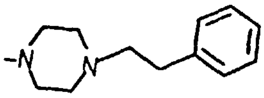 |
| 103 | 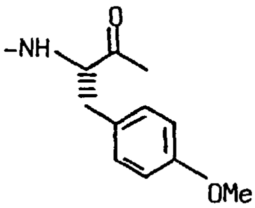 | 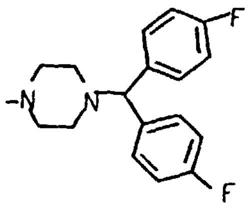 |
| 104 | 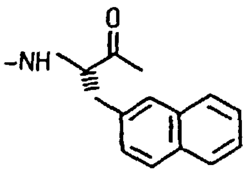 | 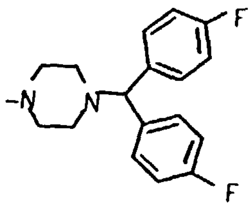 |
| 105 | 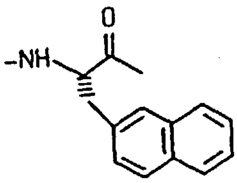 | 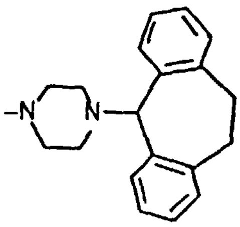 |
| 106 | 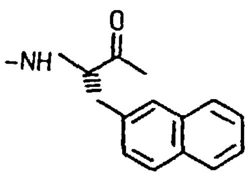 | 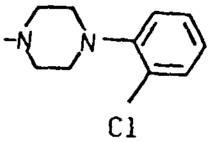 |

| Nr. | $A^2$ | 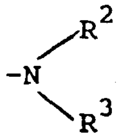 |
| --- | --- | --- |
| 107 | 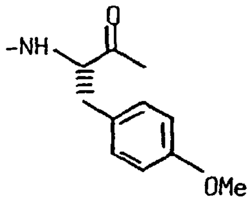 | 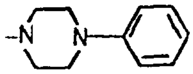 |
| 108 | 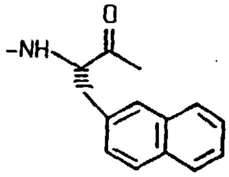 | 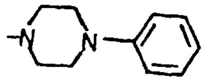 |
| 109 | 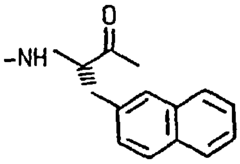 | 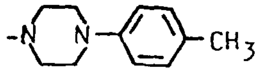 |
| 110 | 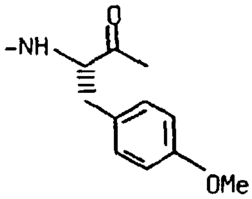 | 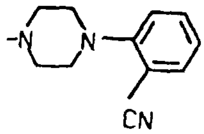 |
| 111 | 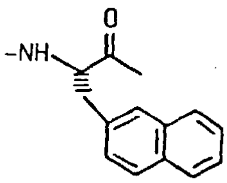 | 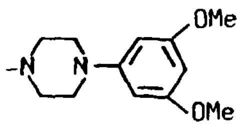 |
| 112 | 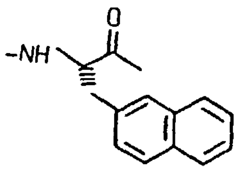 | 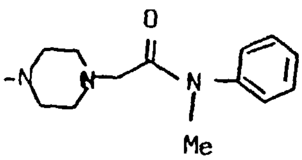 |

| Nr. | $A^2$ | 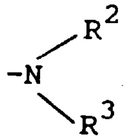 |
|---|---|---|
| 113 | 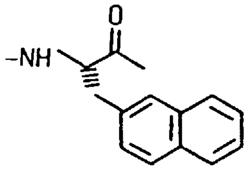 | 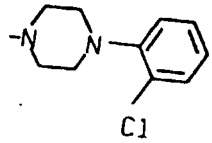 |
| 114 | 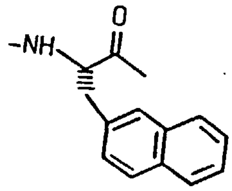 | 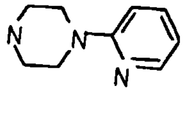 |
| 115 | 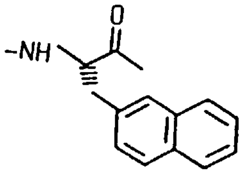 | 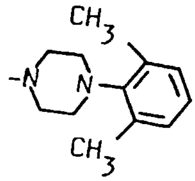 |
| 116 | 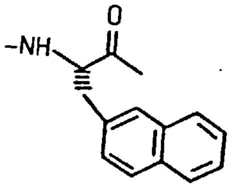 | 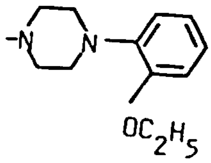 |
| 117 | 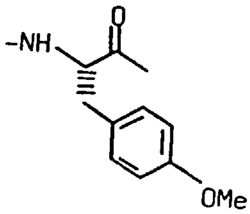 | 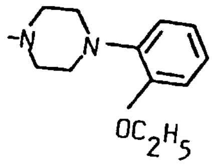 |
| 118 | 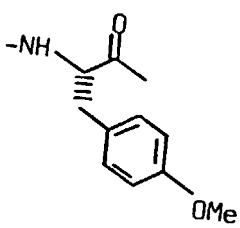 | 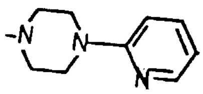 |

| Nr. | $A^2$ | 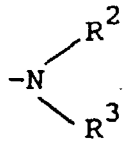 |
|---|---|---|
| 119 | 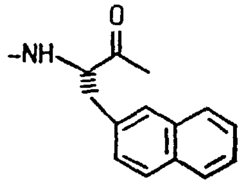 | 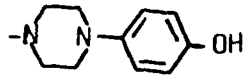 |
| 120 | 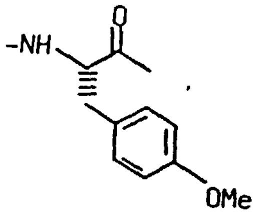 | 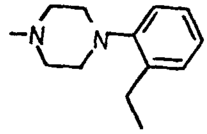 |
| 121 | 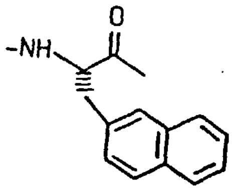 | 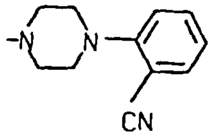 |
| 122 | 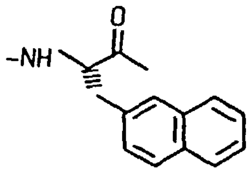 | 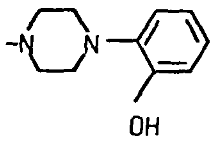 |
| 123 | 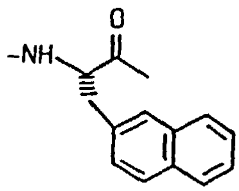 | 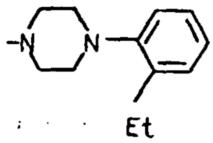 |

| Nr. | $A^2$ | 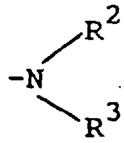 |
| --- | --- | --- |
| 124 | 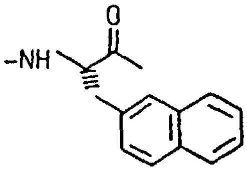 | 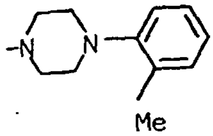 |
| 125 | 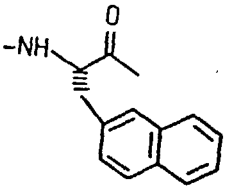 | 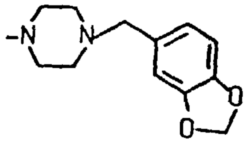 |
| 126 | 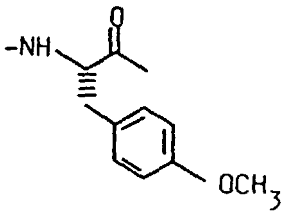 | 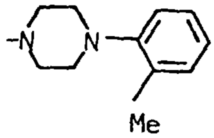 |
| 127 | 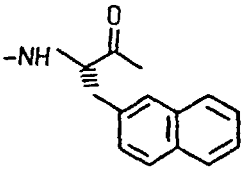 | 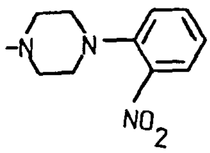 |
| 128 | 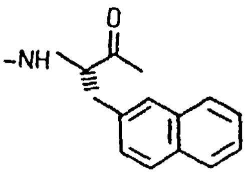 | 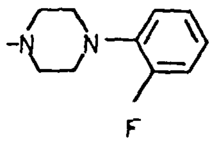 |

| Nr. | $A^2$ | 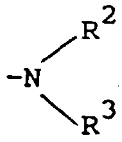 |
|---|---|---|
| 129 | 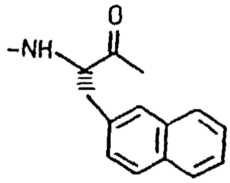 | 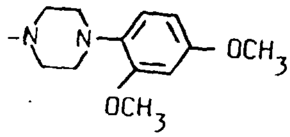 |
| 130 | 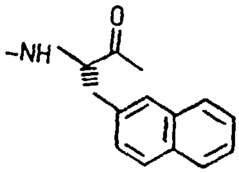 | 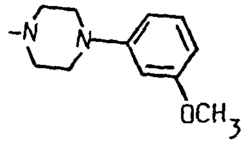 |
| 131 | 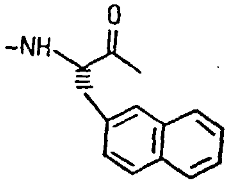 | 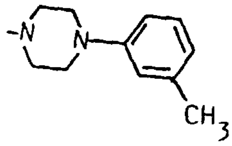 |
| 132 | 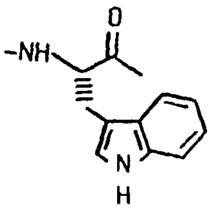 | 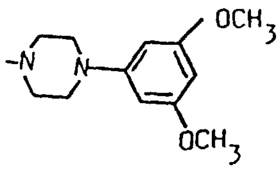 |
| 133 | 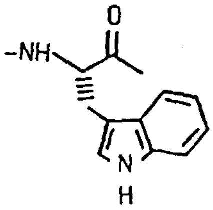 | 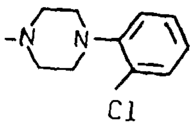 |
| 134 | 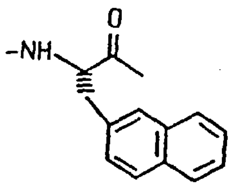 | 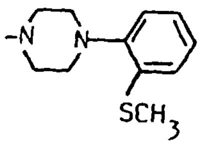 |

Physikalische Daten

Beispiel 1

Fp.: 112-116°C; $[\alpha]_D^{20}$ (MeOH) = -124,4°.

Beispiel 2

Fp.: 70-80°C; $[\alpha]_D^{20}$ (MeOH) = -106,4°.

Beispiel 3

Fp.: 98°C; $[\alpha]_D^{20}$ (MeOH) = -137,9°.

Beispiel 4

Fp.: 115°C; $[\alpha]_D^{20}$ (MeOH) = -120,0°.

Beispiel 5

Fp.: 104-113°C; $[\alpha]_D^{20}$ (MeOH) = -143,4°.

Beispiel 6

Fp.: 96-102°C; $[\alpha]_D^{20}$ (MeOH) = -113,0°.

Beispiel 7

Fp.: 100-106°C; $[\alpha]_D^{20}$ (MeOH) = -130,8°.

Beispiel 8

Fp.: 122-128°C; $[\alpha]_D^{20}$ (MeOH) = -98,0°.

Beispiel 10

Fp.: 95-103°C; $[\alpha]_D^{20}$ (MeOH) = -110,2°.

Beispiel 12

Fp.: 101-109°C; $[\alpha]_D^{20}$ (MeOH) = -117,8°.

Beispiel 14

Fp.: 153-159°C; $[\alpha]_D^{20}$ (MeOH) = -74,4°.

Beispiel 15

Fp.: 102-111°C; $[\alpha]_D^{20}$ (MeOH) = -19,6°.

Beispiel 20

Fp.: 91-103°C; $[\alpha]_D^{20}$ (MeOH) = -120,6°.

Beispiel 22

Fp.: 92-109°C; $[\alpha]_D^{20}$ (MeOH) = -79,4°.

Beispiel 23

Fp.: 73-83°C; $[\alpha]_D^{20}$ (MeOH) = -95,8°.

Beispiel 24

Fp.: 156-165°C; $[\alpha]_D^{20}$ (MeOH) = -52,8°.

Beispiel 28

Fp.: 171-186°C; $[\alpha]_D^{20}$ (MeOH) = -121,6°.

Beispiel 30

Fp.: 75-85°C; $[\alpha]_D^{20}$ (MeOH) = 111,3°.

Beispiel 31

Fp.: 80-90°C; $[\alpha]_D^{20}$ (MeOH) = -111,7°.

Beispiel 32

Fp.: 75-85°C; $[\alpha]_D^{20}$ (MeOH) = -93,8°.

Beispiel 33

Fp.: 105-115°C; $[\alpha]_D^{20}$ (MeOH) = 88,7°.

Beispiel 34

Fp.: 75-85°C; $[\alpha]_D^{20}$ (MeOH) = -65,8°.

Beispiel 35

Fp.: 45-55°C; $[\alpha]_D^{20}$ (MeOH) = -107,0°.

Beispiel 36

Fp.: 45-55°C; $[\alpha]_D^{20}$ (MeOH) = -94,3°.

Beispiel 37

Fp.: 85-95°C; $[\alpha]_D^{20}$ (MeOH) = -75,9°.

Beispiel 38

Fp.: 90-97°C; $[\alpha]_D^{20}$ (MeOH) = -137,5°.

Beispiel 39

Fp.: 80-95°C; $[\alpha]_D^{20}$ (MeOH) = -98,0°.

Beispiel 40

Fp.: 97-105°C; $[\alpha]_D^{20}$ (MeOH) = -66,9°.

Beispiel 41

Fp.: 100-112°C; $[\alpha]_D^{20}$ (MeOH) = -88,6°.

Beispiel 42

Fp.: 105-110°C; $[\alpha]_D^{20}$ (MeOH) = -94,4°.

Beispiel 43

Fp.: 107-115°C; $[\alpha]_D^{20}$ (MeOH) = -108,1°.

Beispiel 44

Fp.: 208-217°C; $[\alpha]_D^{20}$ (MeOH:THF=2:1) = 72,8°.

Beispiel 45

Fp.: ca. 85°(Z.); $[\alpha]_D^{20}$ (MeOH) = -84,8°.

Beispiel 48

Fp.: 104-111°C (Zers.); $[\alpha]_D^{20}$ (MeOH) = -103,1°.

Beispiel 49

Fp.: 118-123°C; $[\alpha]_D^{20}$ (MeOH) = -66,0°.

Beispiel 51

Fp.: 123-128°C; $[\alpha]_D^{20}$ (MeOH) = -111,0°.

Beispiel 55

Fp.: 60-78°C; $[\alpha]_D^{20}$ (MeOH) = -103,7°.

Beispiel 57

Fp.: 61-64°C; $[\alpha]_D^{20}$ (MeOH) = -107,6°.

Beispiel 58

Fp.: 55-65°C; $[\alpha]_D^{20}$ (MeOH) = 76,1°.

Beispiel 59

Fp.: 85-89°C; $[\alpha]_D^{20}$ (MeOH) = -118,6°.

Beispiel 60

Fp.: 45-56°C; $[\alpha]_D^{20}$ (MeOH) = -78,1°.

Beispiel 61

Fp.: 68-72°C; $[\alpha]_D^{20}$ (MeOH) = -108,2°.

Beispiel 62

Fp.: 56-60°C; $[\alpha]_D^{20}$ (MeOH) = -47,0°.

Beispiel 68

Fp.: - (glasig); $[\alpha]_D^{20}$ (MeOH) = -58,1°.

Beispiel 70

Fp.: 66-76°C; $[\alpha]_D^{20}$ (MeOH) = -37,6°.

Beispiel 71

Fp.: -(pastös); $[\alpha]_D^{20}$ (MeOH) = -50,2°.

Beispiel 73

FAB-MS: $(M+H)^+$ 640,2

Beispiel 74

Fp.: 93-95°C; FAB-MS: $(M+H)^+$ 638,4

Beispiel 75

Fp.: 67-70°C; FAB-MS: $(M+H)^+$ 640,4

Beispiel 76

Fp.: über 200°C; FAB-MS: $(M+H)^+$ 638,3

Beispiel 77

Fp.: 133-138°C; FAB-MS: $(M+H)^+$ 698,4

Beispiel 78

Fp.: über 200°C; FAB-MS: $(M+H)^+$ 640,3

Beispiel 79

Fp.: 109-114°C; FAB-MS: $(M+H)^+$ 668,4

Beispiel 80

Fp.: 142-146°C; FAB-MS: $(M+H)^+$ 668,4

Beispiel 81

Fp.: 109-115°C; FAB-MS: $(M+H)^+$ 640,5

Beispiel 82

Fp.: 145-152°C schäumt; FAB-MS: $(M+H)^+$ 655,3

Beispiel 83

Fp.: 110-115°C; FAB-MS: $(M+H)^+$ 655,3

Beispiel 84

Fp.: 144-150°C; FAB-MS: $(M+H)^+$ 698,4

Beispiel 85

Fp.: 115-122°C; FAB-MS: $(M+H)^+$ 712,4

Beispiel 86

Fp.: 132-140°C; FAB-MS: $(M+H)^+$ 712,4

Beispiel 87

Fp.: 144-149°C; FAB-MS: $(M+H)^+$ 718,4

Beispiel 88

FAB-MS: $(M+H)^+$ 714,4

Beispiel 89

Fp.: 140-144°C; FAB-MS: $(M+H)^+$ 720,3

Beispiel 91

Fp.: 110-117°C; FAB-MS: $(M+H)^+$ 700,4

Beispiel 92

Fp.: 101-108°C; FAB-MS: $(M+H)^+$ 616,5

Beispiel 93

Fp.: 138-143°C; FAB-MS: $(M+H)^+$ 700,4

Beispiel 94

Fp. Base: 126-134°C; FAB-MS: $(M+H)^+$ 660,3
HU-Salz 174-178°C

Beispiel 95

Fp.: 143-148°C; FAB-MS: $(M+H)^+$ 732,3

Beispiel 96

Fp.: 135-142°C schäumt; FAB-MS: $(M+H)^+$ 734,3

Beispiel 97

Fp.: 140-144°C; FAB-MS: $(M+H)^+$ 688,3

Beispiel 98

FAB-MS: $(M+H)^+$ 714,3

Beispiel 99

FAB-MS: $(M+H)^+$ 636,3

Beispiel 100

Fp.: 143-150°C; FAB-MS: $(M+H)^+$ 675,2

Beispiel 101

Fp.: 122-128°C; FAB-MS: $(M+H)^+$ 660,1

Beispiel 102

FAB-MS: $(M+H)^+$ 658,5

Beispiel 103

FAB-MS: $(M+H)^+$ 736,3

Beispiel 104

FAB-MS: $(M+H)^+$ 756,3

Beispiel 105

Fp.: 142-148°C schäumt; FAB-MS: $(M+H)^+$ 746,6

Beispiel 106

Fp.: 137-145°C;

Beispiel 107

Fp.: 124-133°C; FAB-MS: $(M+H)^+$ 610,5

Beispiel 108

Fp.: 156-159°C; FAB-MS: $(M+H)^+$ 630,5

Beispiel 109

Fp.: 206-211°C;

Beispiel 110

FAB-MS; $(M+H)^+$ 635,3

Beispiel 111

Fp.: 125-128°C schäumt; FAB-MS: $(M+H)^+$ 690,5

Beispiel 112

Fp.: 138-140°C schäumt; FAB-MS: $(M+H)^+$ 701,5

Beispiel 113

Fp.: 201-203°C;

Beispiel 114

Fp.: 144-147°C; FAB-MS: $(M+H)^+$ 631,3

Beispiel 115

Fp.: 134-139°C; FAB-MS: $(M+H)^+$ 658,3

Beispiel 116

Fp.: 130-133°C; FAB-MS: $(M+H)^+$ 674,5

Beispiel 117

Fp.: 110-115°C; FAB-MS: $(M+H)^+$ 654,5

Beispiel 118

Fp.: 107-112°C schäumt; FAB-MS: $(M+H)^+$ 611,4

Beispiel 119

Fp.: 159-162°C; FAB-MS: $(M+H)^+$ 646,3

Beispiel 120

Fp.: 117-122°C; FAB-MS: $(M+H)^+$ 638,3

Beispiel 122

Fp.: 148-152°C; FAB-MS: $(M+H)^+$ 646,3

Beispiel 123

Fp.: 128-132°C; FAB-MS: $(M+H)^+$ 658,4

Beispiel 124

FAB-MS: $(M+H)^+$ 644,5

Beispiel 125

FAB-MS: $(M+H)^+$ 674

Beispiel 126

FAB-MS: $(M+H)^+$ 623,4

Tabelle 5

**Beispiele 135 - 157**

Beispiel 135

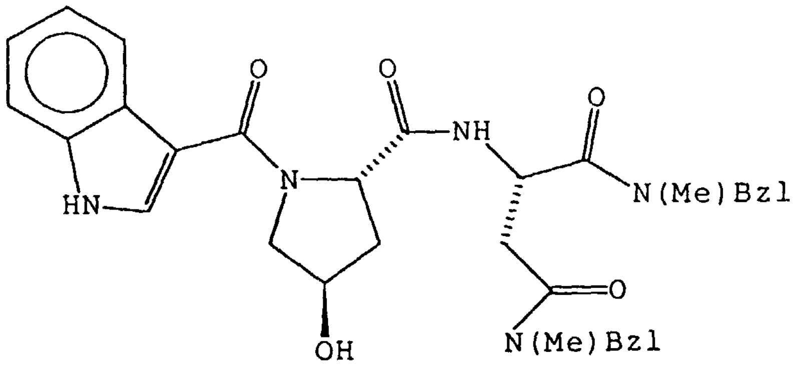

Fp.:80-88°C

$[\alpha]_D^{20}$ (MeOH) = -134,4°

Beispiel 136

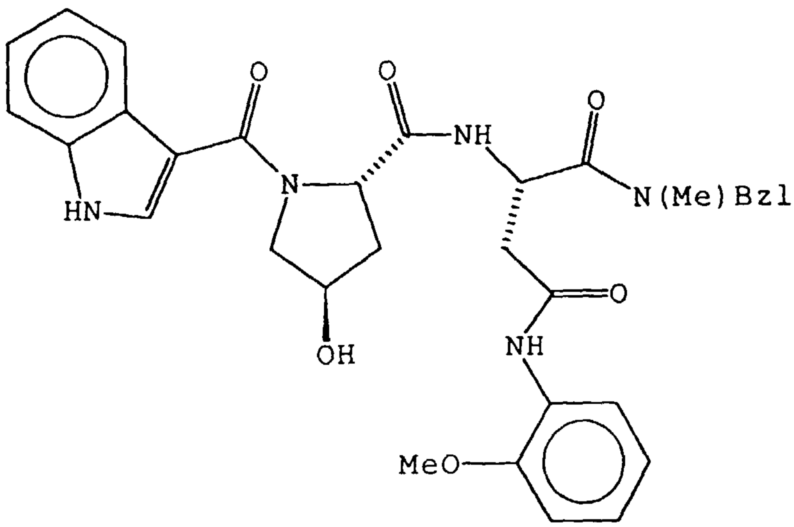

Fp.:128-136°C

$[\alpha]_D^{20}$ (MeOH) = -137,5°

Beispiel 137

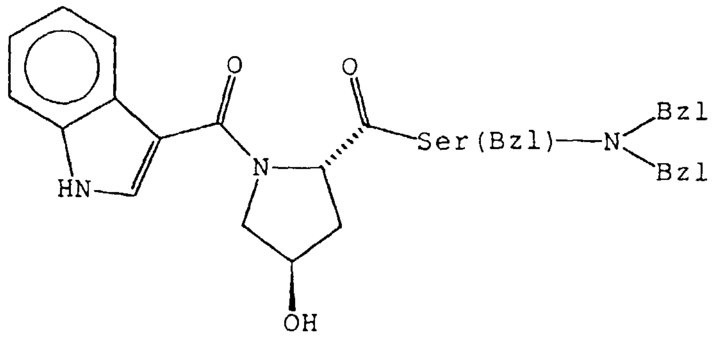

Fp.: 85-95°C

$[\alpha]_D^{20}$(MeOH) = -79,6°

Beispiel 138

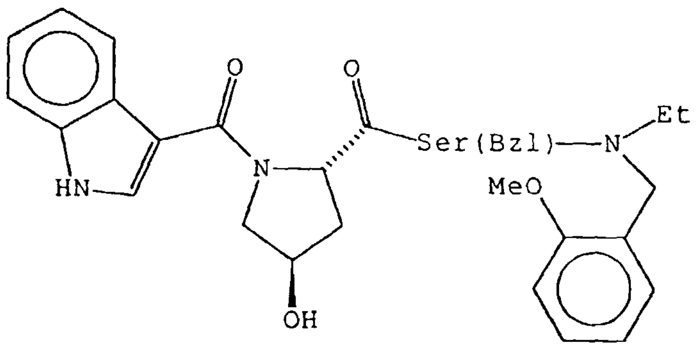

Fp.: 77-82°C

$[\alpha]_D^{20}$(MeOH) = -103,4°

Beispiel 139

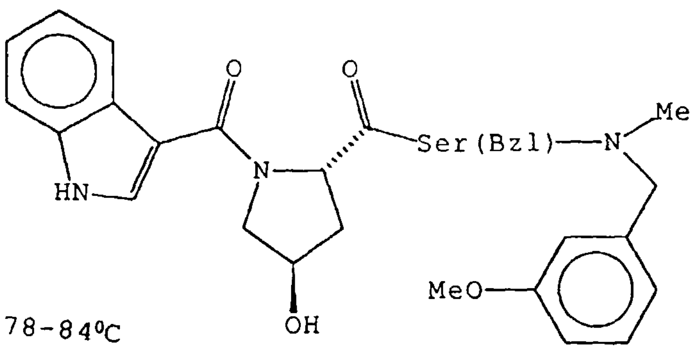

Fp.: 78-84°C

$[\alpha]_D^{20}$(MeOH) = -97,0°

Beispiel 140

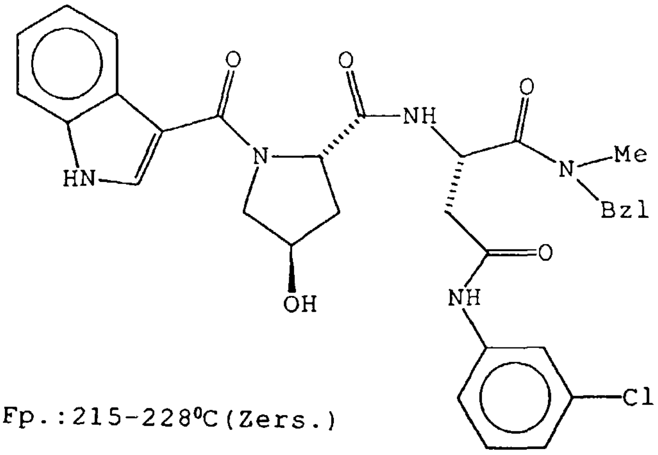

Fp.:215-228°C(Zers.)

Beispiel 141

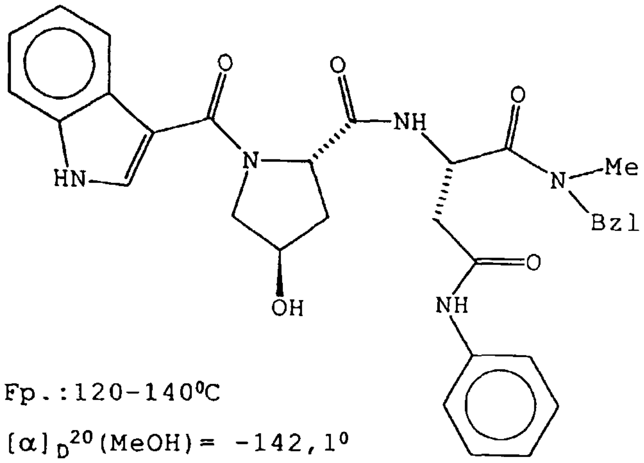

Fp.:120-140°C

$[\alpha]_D^{20}$(MeOH)= -142,1°

Beispiel 142

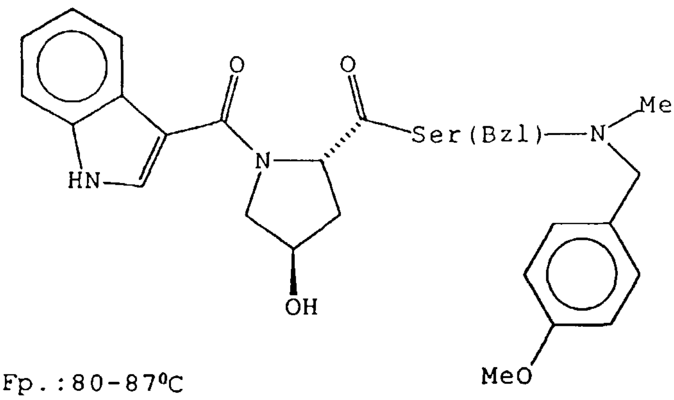

Fp.:80-87°C

$[\alpha]_D^{20}$(MeOH)= -95,2°

Beispiel 143

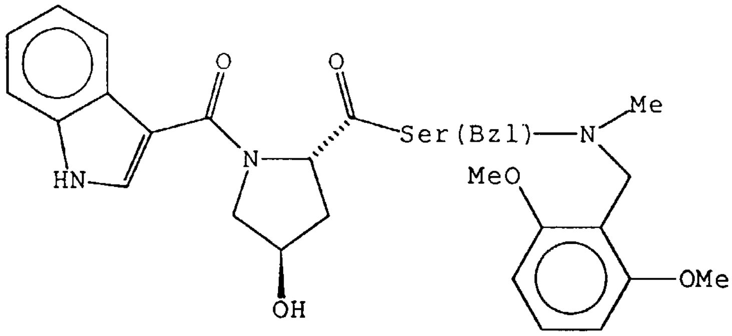

Fp.: 84-88°C

$[\alpha]_D^{20}$(MeOH) = -113,0°

Beispiel 144

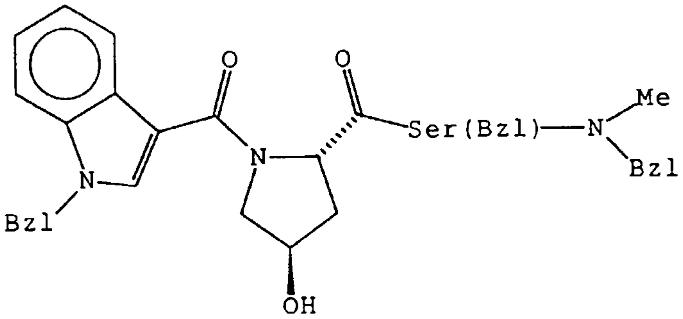

Fp.: ca. 80°C (Zers.)

$[\alpha]_D^{20}$(MeOH) = -82,4°

Beispiel 145

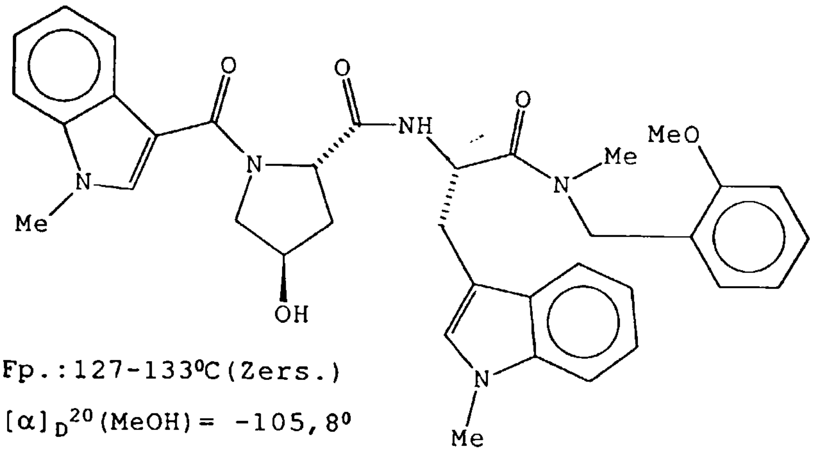

Fp.: 127-133°C (Zers.)

$[\alpha]_D^{20}$(MeOH) = -105,8°

72

Beispiel 146

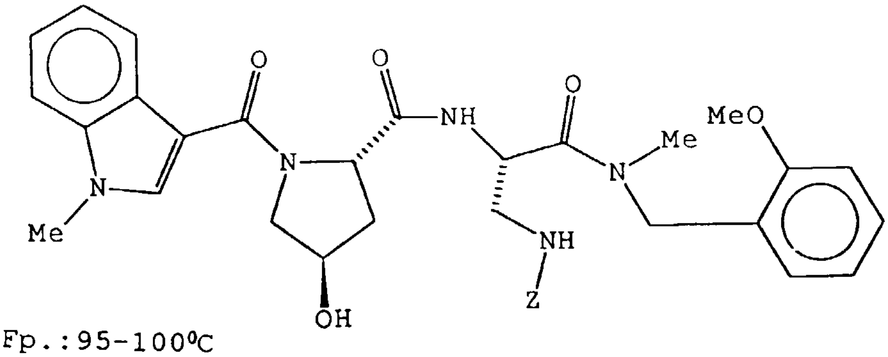

Fp.: 95-100°C

$[\alpha]_D^{20}$(MeOH) = -111,5°.

Beispiel 147

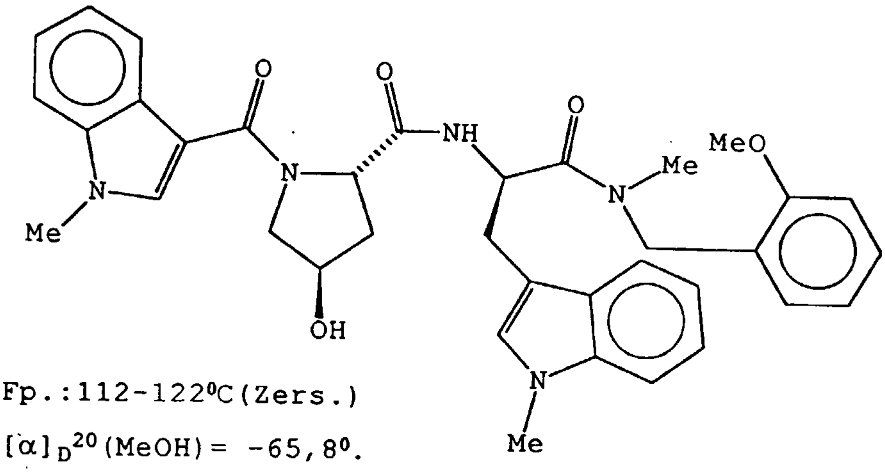

Fp.: 112-122°C (Zers.)

$[\alpha]_D^{20}$(MeOH) = -65,8°.

Beispiel 148

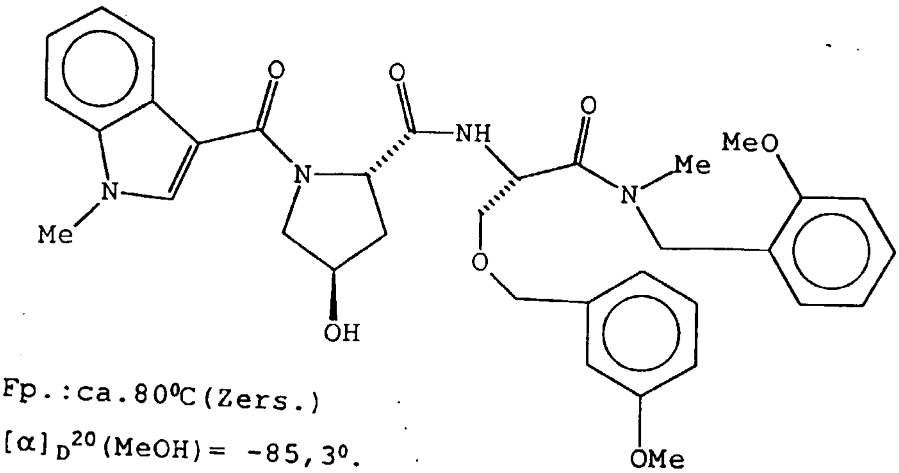

Fp.: ca. 80°C (Zers.)

$[\alpha]_D^{20}$(MeOH) = -85,3°.

Beispiel 149

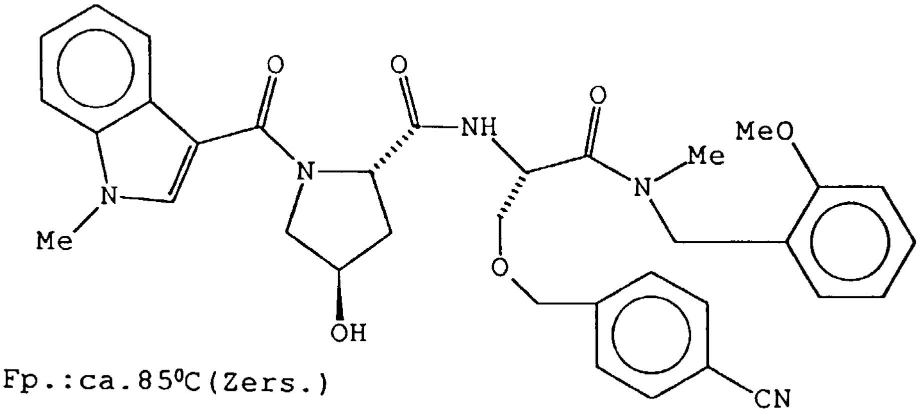

Fp.: ca. 85°C (Zers.)

$[\alpha]_D^{20}$ (MeOH) = -90,6°.

Beispiel 150

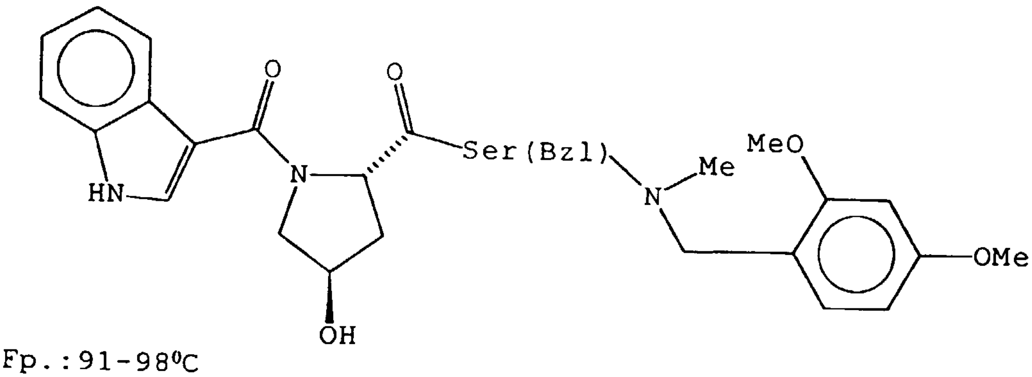

Fp.: 91-98°C

$[\alpha]_D^{20}$ (MeOH) = -112,4°

Beispiel 151

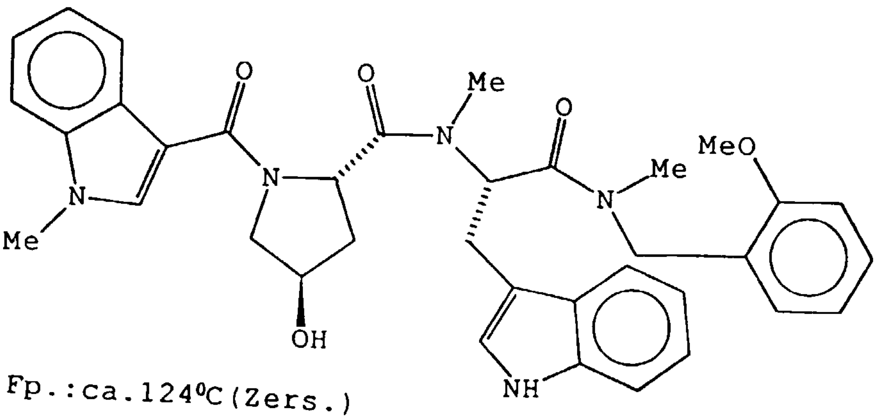

Fp.: ca. 124°C (Zers.)

$[\alpha]_D^{20}$ (MeOH) = -151,5°

Beispiel 152

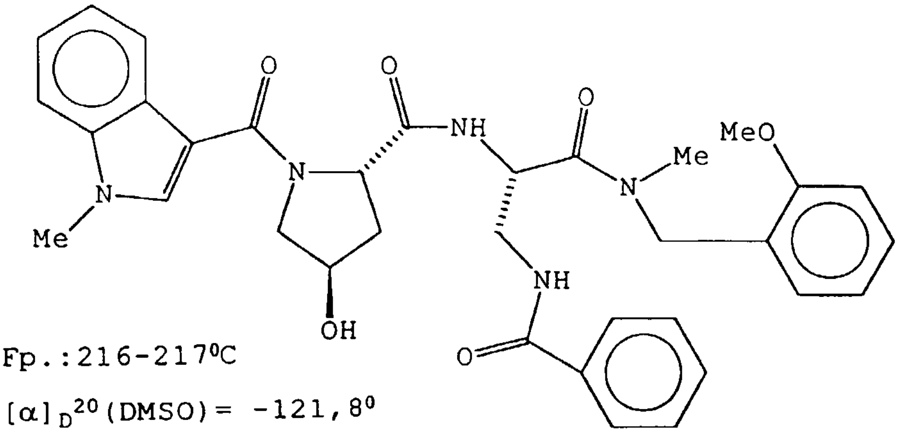

Fp.:216-217°C

$[\alpha]_D^{20}$(DMSO)= -121,8°

Beispiel 153

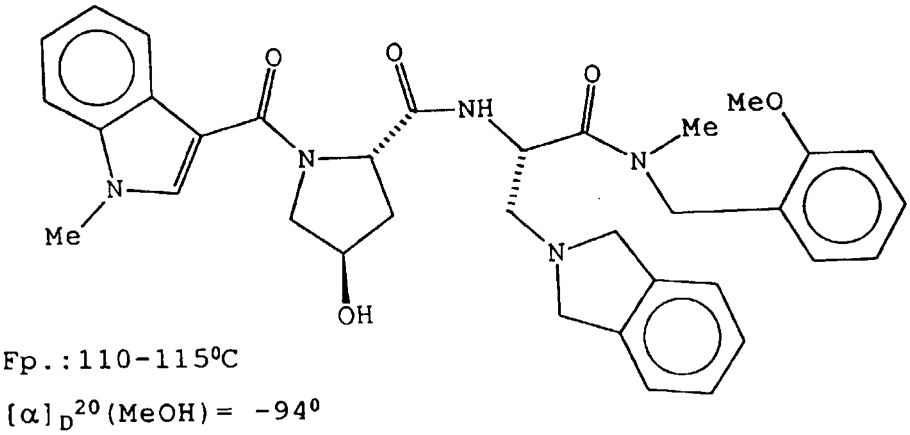

Fp.:110-115°C

$[\alpha]_D^{20}$(MeOH)= -94°

Beispiel 154

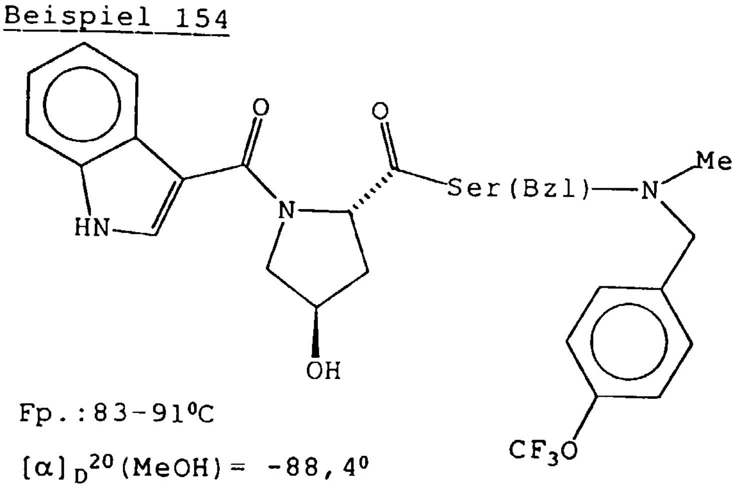

Fp.:83-91°C

$[\alpha]_D^{20}$(MeOH)= -88,4°

Beispiel 155

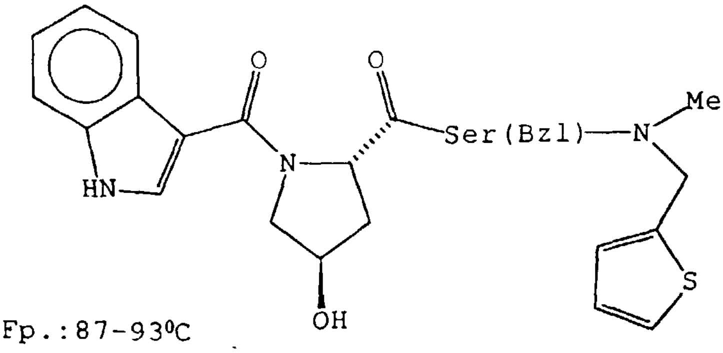

Fp.: 87-93 °C

$[\alpha]_D^{20}$ (MeOH) = -105,4°

Beispiel 156

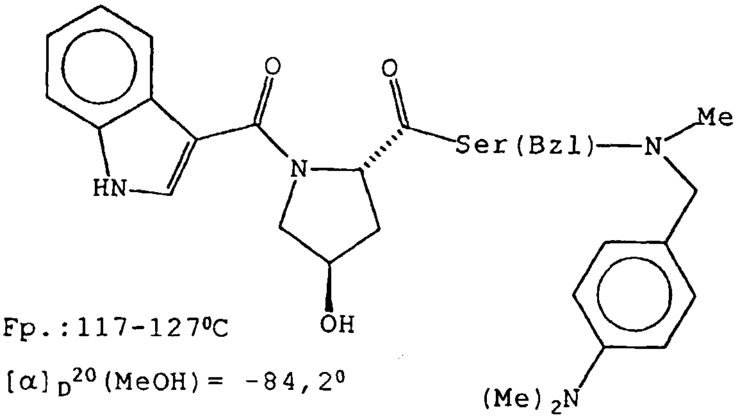

.HCl

Fp.: 117-127 °C

$[\alpha]_D^{20}$ (MeOH) = -84,2°

Beispiel 157

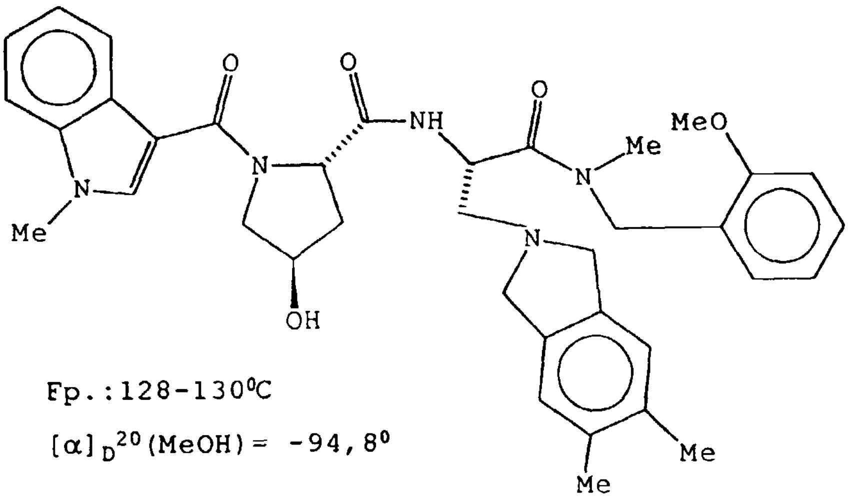

Fp.: 128-130 °C

$[\alpha]_D^{20}$ (MeOH) = -94,8°

Tabelle 6

**Beispiele 158 - 182**

Beispiel 158

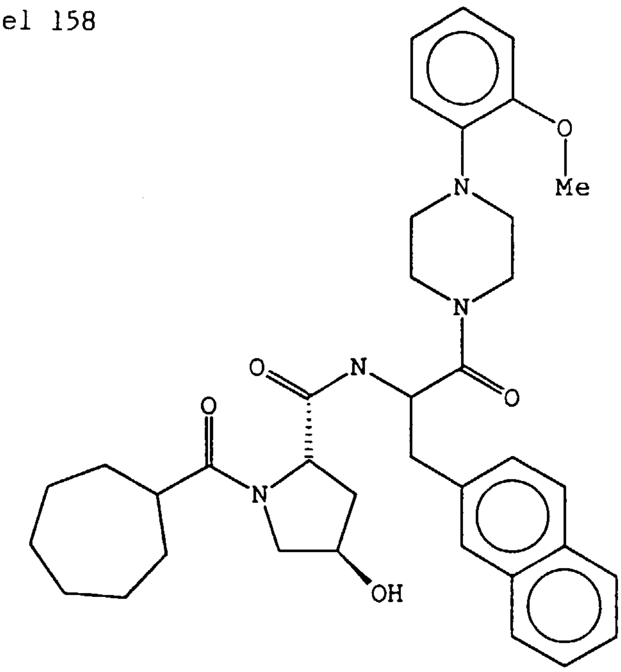

.HCl

Beispiel 159

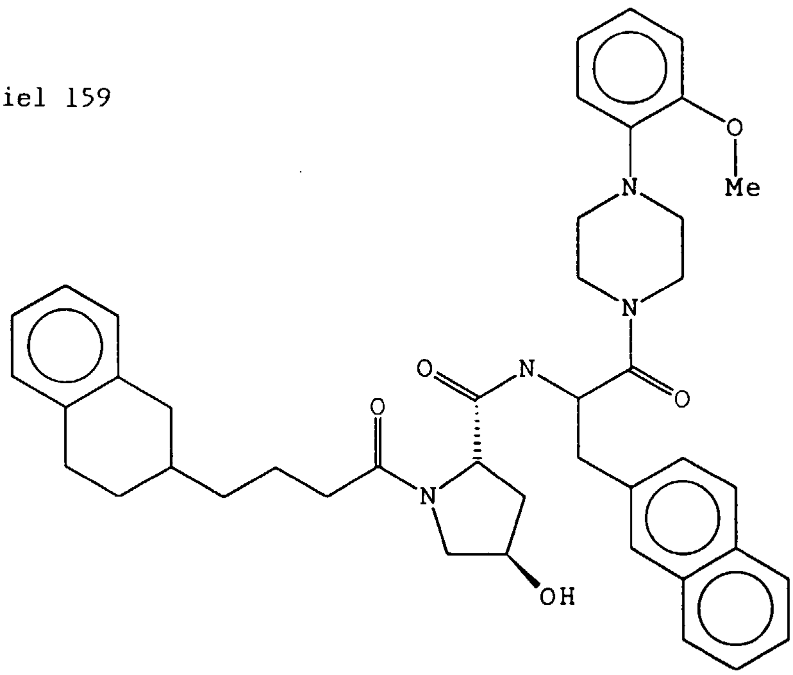

.HCl

Beispiel 160

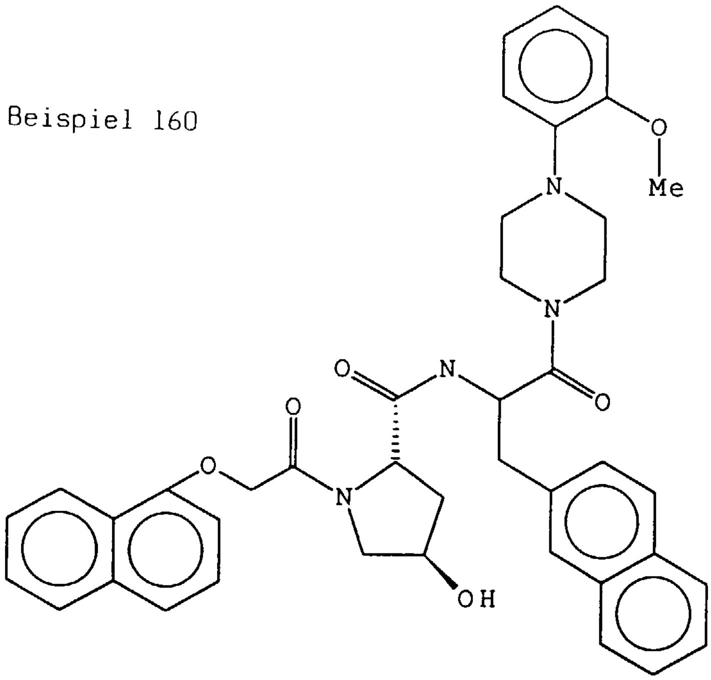

.HCl

Beispiel 161

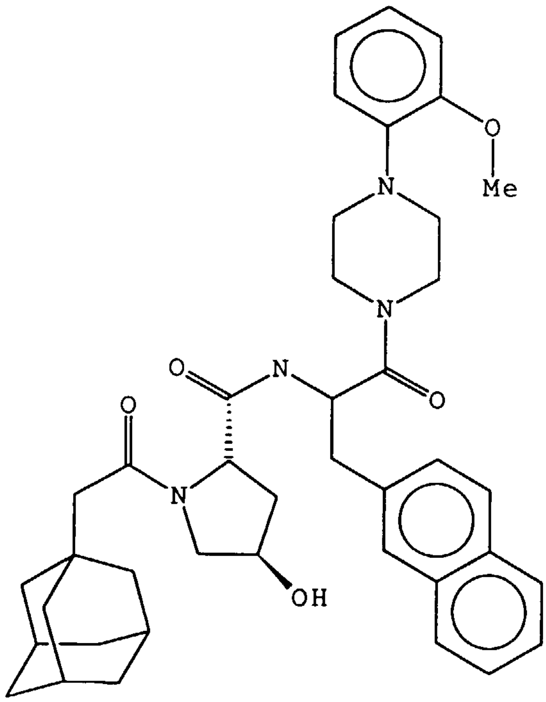

.HCl

Beispiel 162

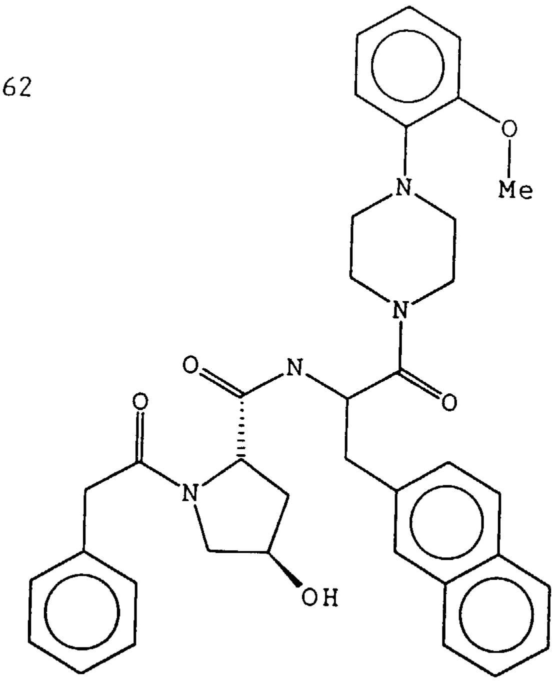

.HCl

Beispiel 163

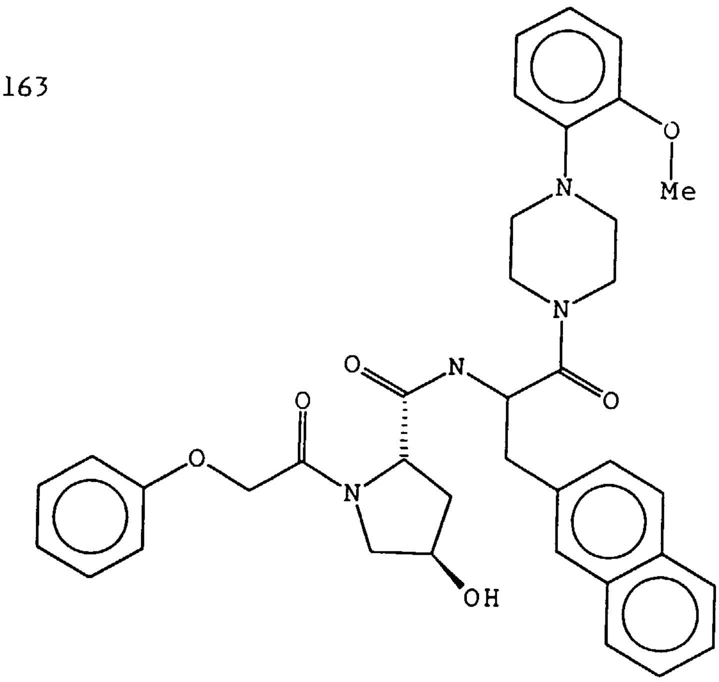

.HCl

Beispiel 164

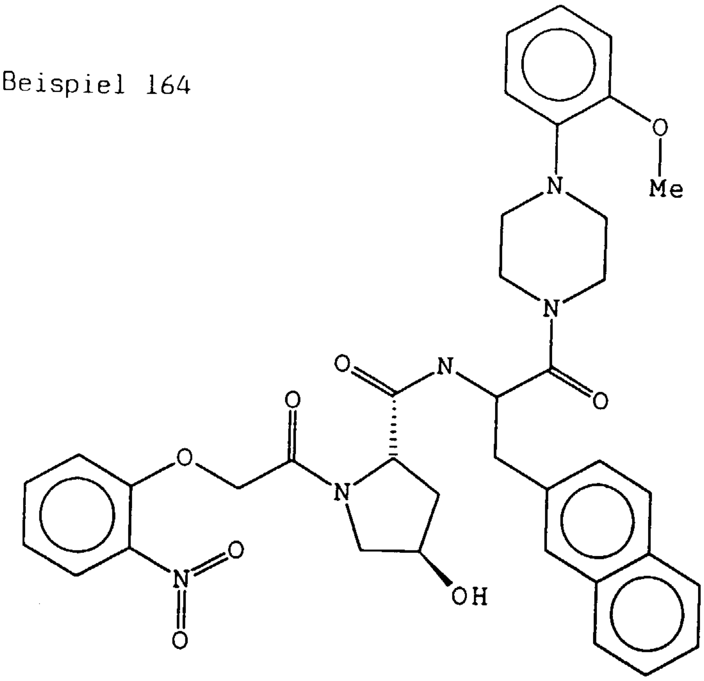

.HCl

Beispiel 165

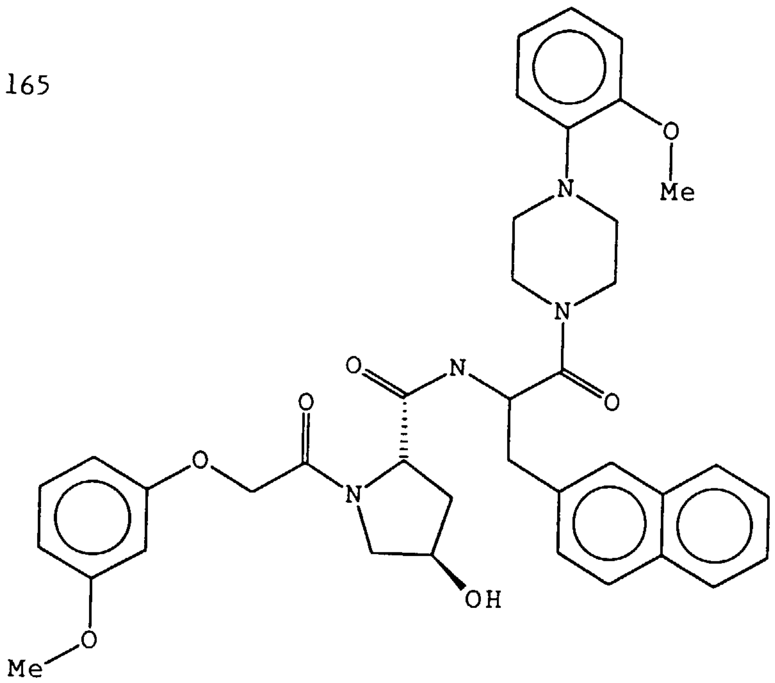

.HCl

Beispiel 166

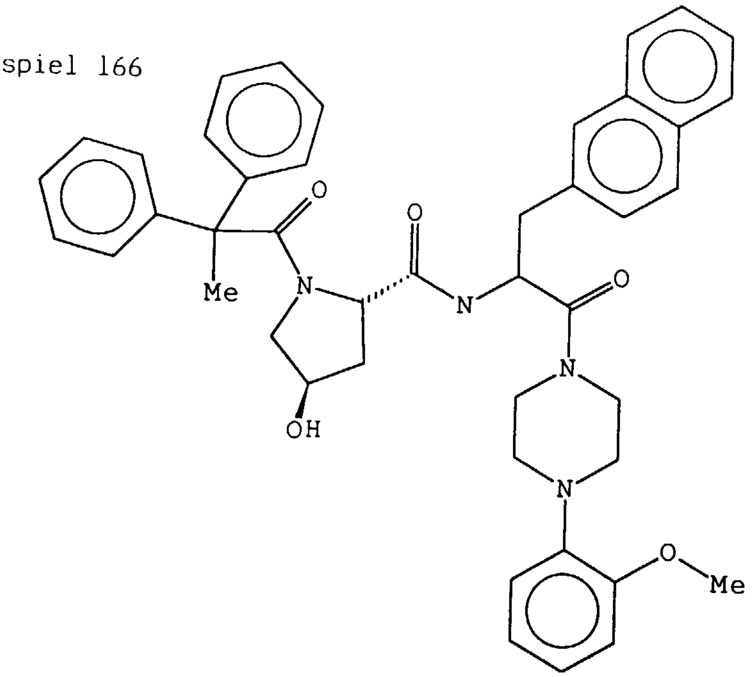

.HCl

Beispiel 167

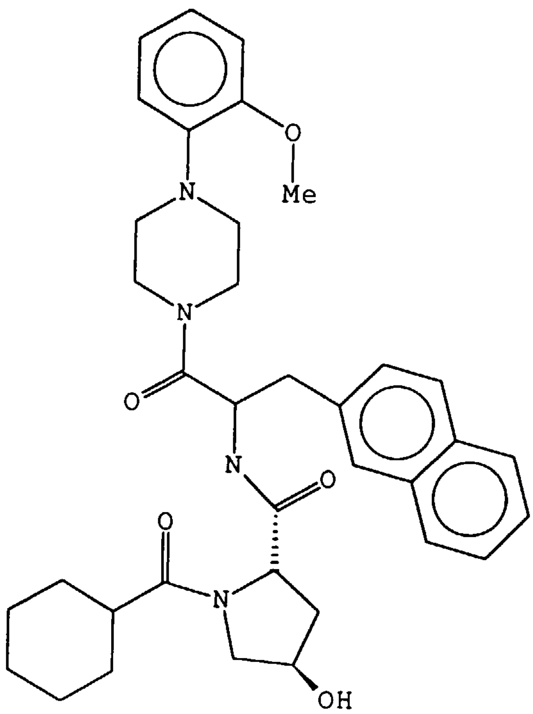

.HCl

Beispiel 166

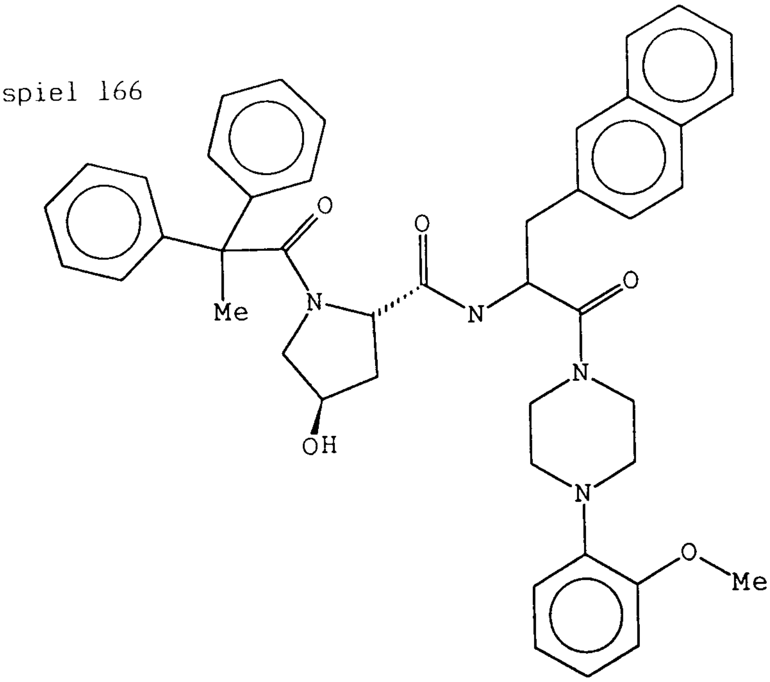

.HCl

Beispiel 167

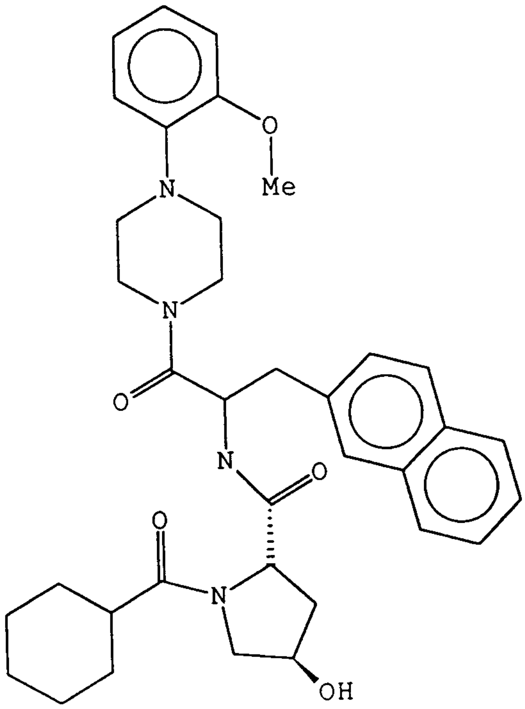

.HCl

Beispiel 170

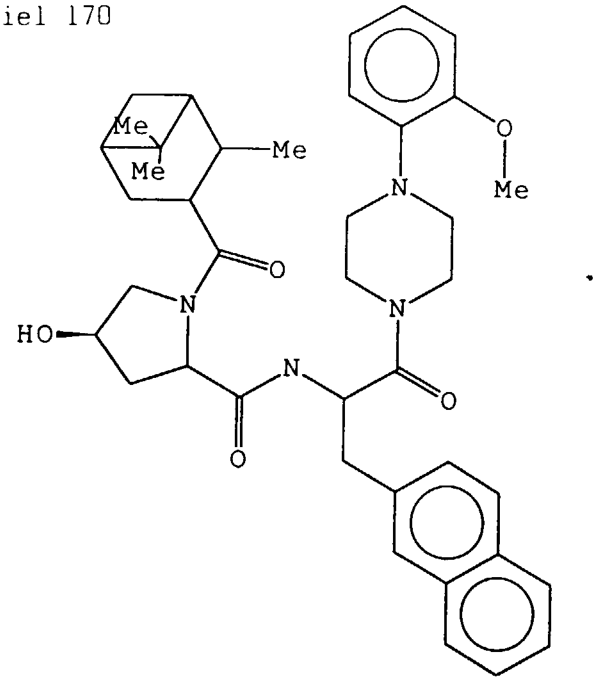

.HCl

Beispiel 171

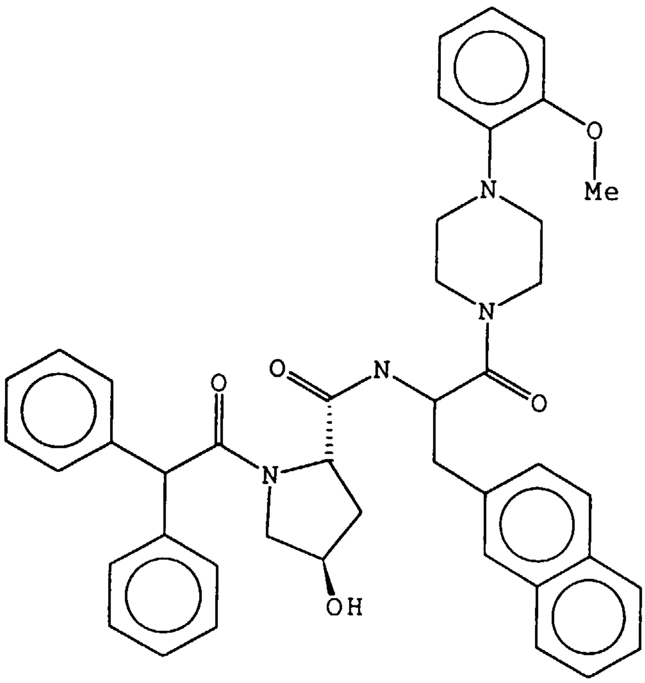

.HCl

Beispiel 172

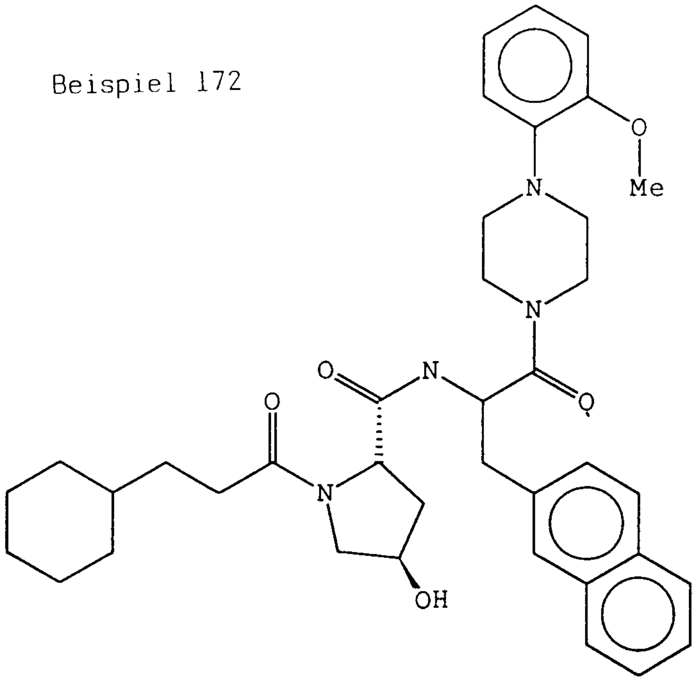

.HCl

Beispiel 173

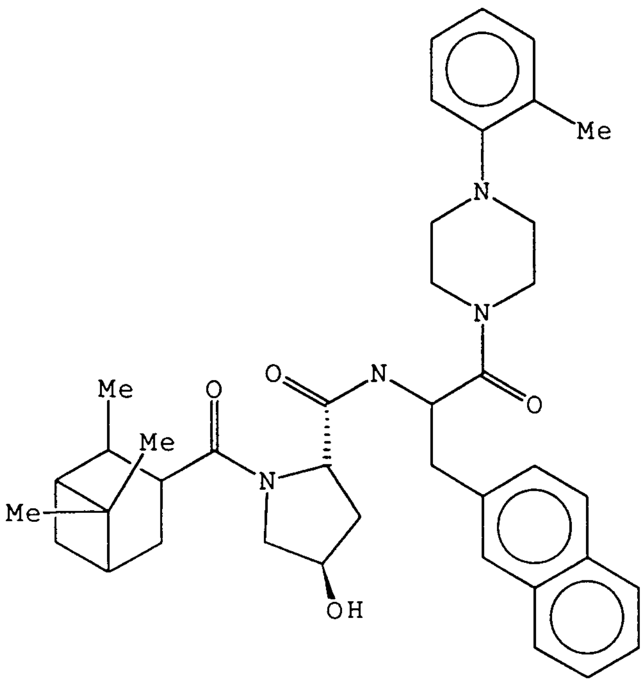

.HCl

Beispiel 174

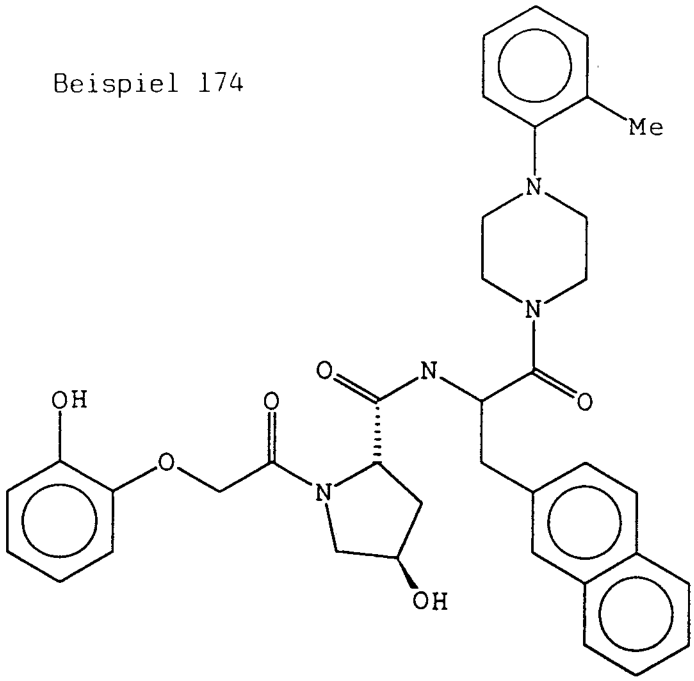

.HCl

Beispiel 175

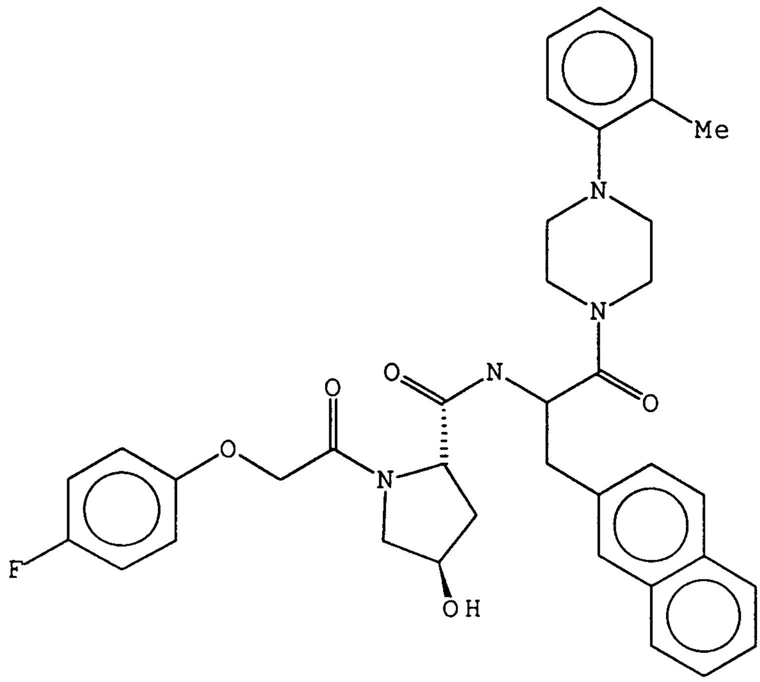

.HCl

Beispiel 176

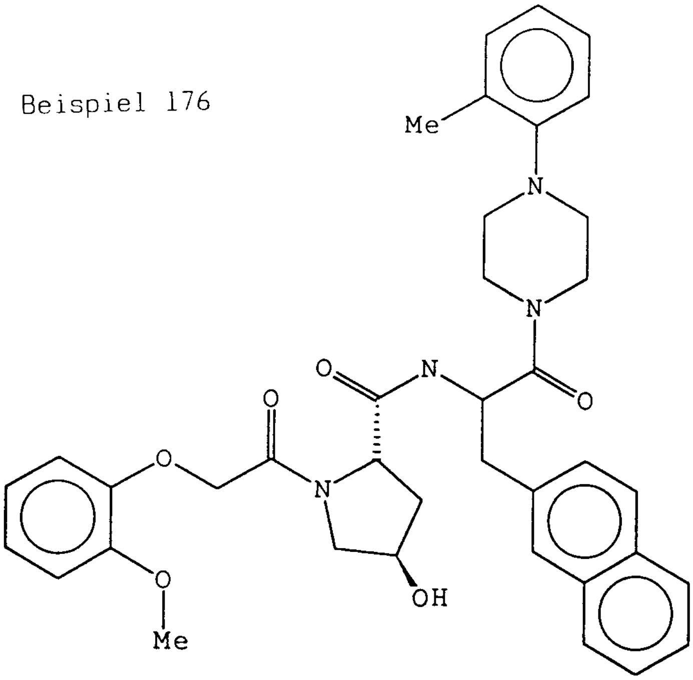

.HCl

Beispiel 177

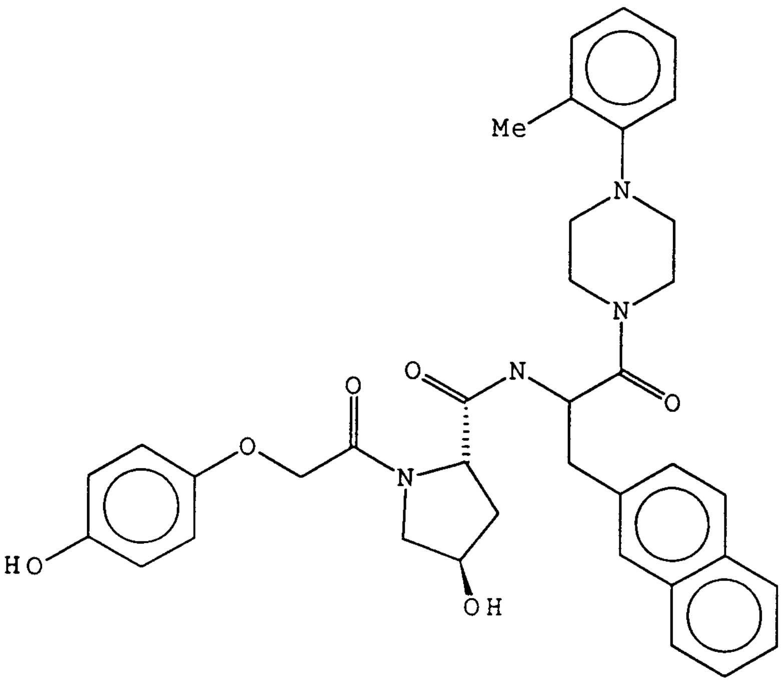

.HCl

Beispiel 178

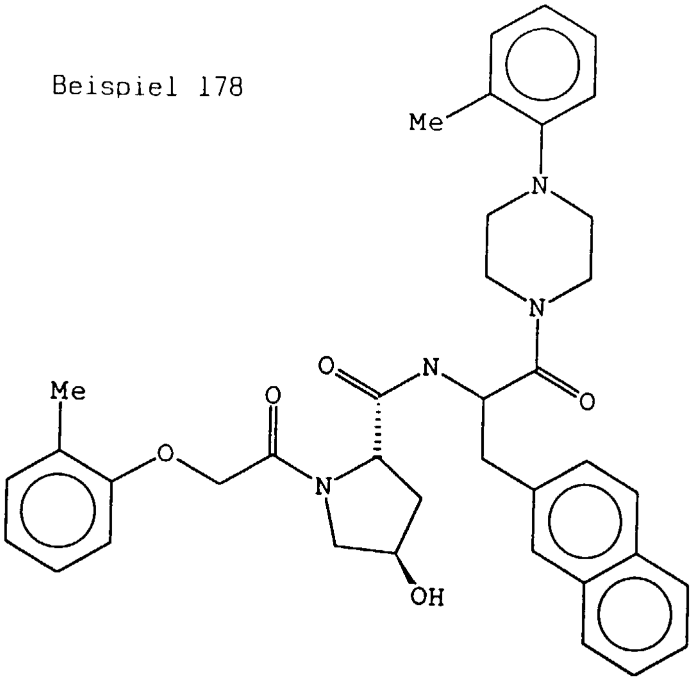

.HCl

Beispiel 179

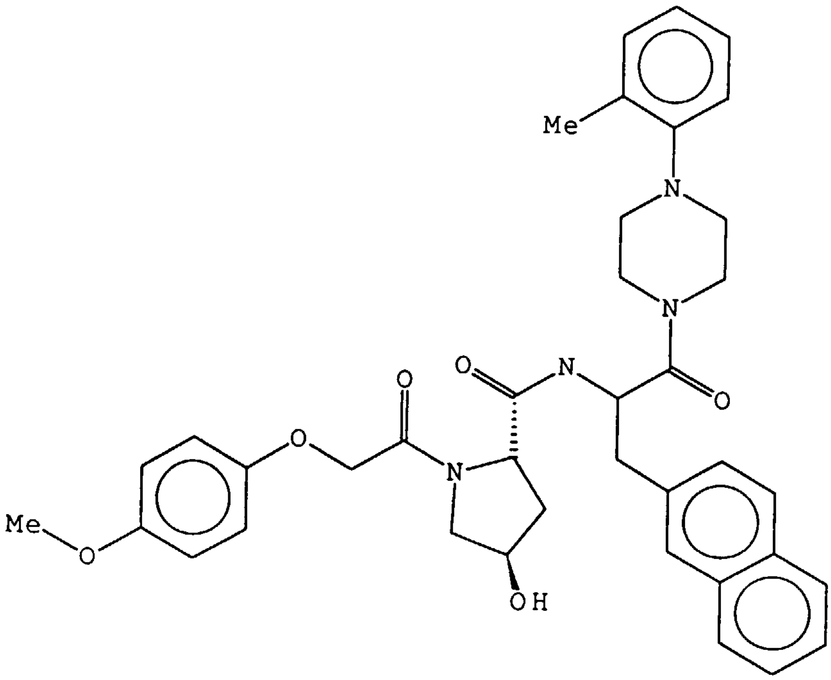

.HCl

Beispiel 180

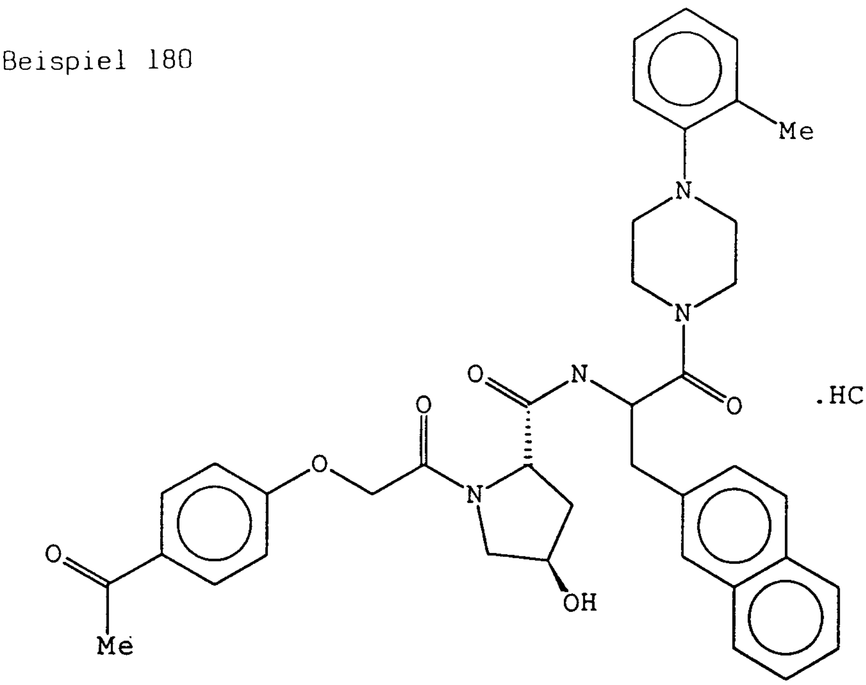

.HCl

Beispiel 181

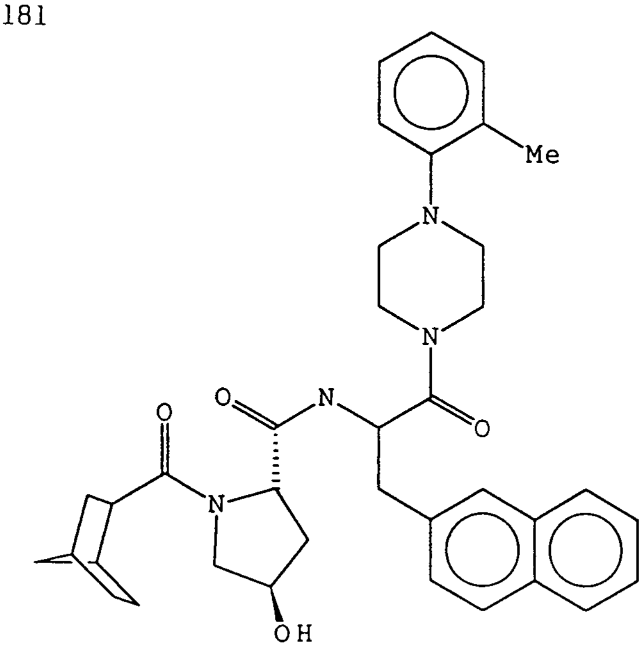

.HCl

Beispiel 182

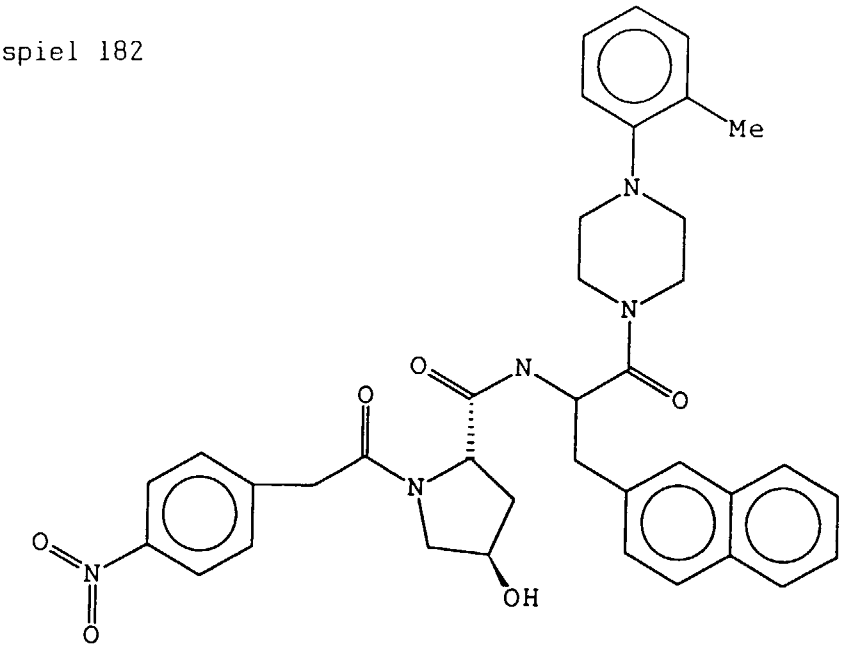

.HCl

| Physikalische Daten | |
|---|---|
| Beispiel Nr. | FAB MS: $(M+H)^+$ |
| 158 | 627,5 |
| 159 | 703,5 |
| 160 | 687,4 |
| 161 | 679,5 |
| 162 | 621,3 |
| 163 | 637,3 |
| 164 | 682,3 |
| 165 | 667,2 |
| 166 | 711,3 |
| 167 | 613,3 |
| 168 | 585,2 |
| 169 | 599,4 |
| 170 | 667,3 |
| 171 | 697,4 |
| 172 | 641,4 |
| 173 | 651,5 |
| 174 | 637,3 |
| 175 | 639,3 |
| 176 | 651 |

(fortgesetzt)

| Physikalische Daten | |
|---|---|
| Beispiel Nr. | FAB MS: $(M+H)^+$ |
| 177 | 637 |
| 178 | 635 |
| 179 | 651 |
| 180 | 663,1 |
| 181 | 608,4 |
| 182 | 650,3 |

## Patentansprüche

**1.** Aminosäurederivat der allgemeinen Formel I

$$R^1 - \overset{O}{\overset{\|}{C}} - A^1 - A^2 - NR^2R^3 \qquad (I)$$

oder dessen pharmazeutisch annehmbares Salz, worin

$R^1$ Vinyl, Aryl, Heteroaryl, Aralkyl, Heteroaralkyl, Arylvinyl, Heteroarylvinyl, Aryloxyalkyl, Arylalkyloxy, $(C_3-C_8)$ Cycloalkyl, $(C_3-C_8)$Cycloalkylalkyl, unsubsituiertes oder durch 1-3 Methylgruppen substituiertes Bicycloheptyl oder Bicycloheptylalkyl, Adamantyl, Adamantylalkyl, Dekalin, Dekalinalkyl, Tetralin, Tetralinalkyl, Diphenylalkyl, Di(arylalkyl)aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl, Hydroxy, Nitro, Alkylcarbonyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin ($\Delta$Pro) wie beispielsweise 3,4-Didehydroprolin ($\Delta$(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L-Aminoprolin (Pro($NH_2$)) wie beispielsweise 3-Aminoprolin (Pro(3$NH_2$)) und 4-Aminoprolin (Pro(4$NH_2$)), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), Tpr($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$A^2$ eine lipophile $\alpha$-Aminosäure ist, die eine Phenyl-, 1-, 2- oder 3-fach substituierte Phenyl-, Heteroaryl-, Cyclohexyl- oder Cyclopentylgruppe, eine Naphthylgruppe oder eine Mono- oder Di-$C_{1-3}$-alkylaminogruppe enthält, und diese Ringgruppe bzw. Aminogruppe durch eine 1- bis 8-gliedrige Kette vom Backbone der Aminosäure getrennt ist, (wobei die Substituenten der Phenylgruppe unabhängig voneinander Halogen, Trihalogenomethyl, Alkoxy, Alkyl, Cyano oder 1-Pyrrolidinyl sind und wobei in der 1- bis 8-gliedrigen Kette die Glieder der Kette $-CHR^4$, -C(O)-, -O-, -S- und/oder $-NR^4$-sein können, die so angeordnet sind, daß sich eine der folgenden 3 Kettenarten ergibt

$-(CHR^4)_{1-8}-$
$-(CHR^4)_{0-p}-G^1-(CHR^4)_{0-q}-$
$-(CHR^4)_{1-p}-G^2-(CHR^4)_{0-q}-$

worin $G^1$ -C(O)O- oder -C(O)-$NR^4$- ist, $G^2$ -O-, -S-, -$NR^4$-C(O)-O-, -$NR^4$-C(O)-, -$NR^4$-C(O)-$NR^4$- oder -O-C(O)-$NR^4$- ist und p und q ganze Zahlen von 1 bis 6 sind, die so gewählt werden, daß die Gesamtzahl der Kettenglieder 1 bis 8 ist,
und $R^4$ für Wasserstoff, Alkyl, Aryl oder Aralkyl steht, wobei

Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl oder Cyano sind; und die Alkylgruppe 1 bis 3 Kohlenstoffatome enthält; (wobei, wenn eine Kette mehr als eine -$CHR^4$-Gruppe enthält, nur in einer dieser -$CHR^4$-Gruppen $R^4$ Alkyl, Aryl oder Aralkyl sein kann und
wobei die Kette nicht $CH_2$ ist, wenn $R^2$ und $R^3$ Alkyl oder Aralkyl sind und wenn

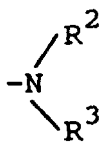

zusammen die Gruppe

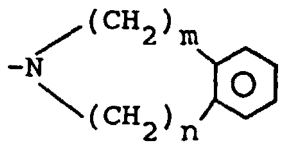

sind und m und n je 0, 1, 2 oder 3 sind, wobei deren Summe 2, 3, 4 oder 5 ergibt) oder $A^2$ Leu, Ile, Nle, Val, Met

oder eine der Gruppen

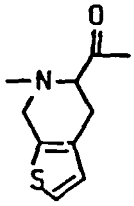 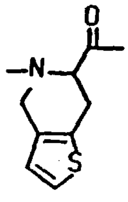 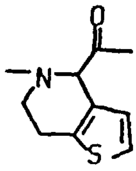 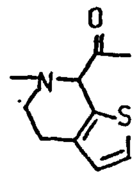

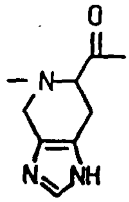 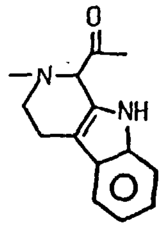 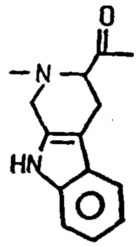

**und** 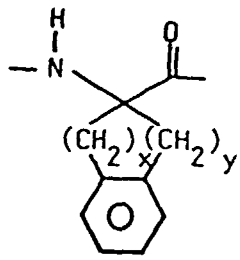

(worin x und y unabhängig voneinander 1 oder 2 sind) ist;

$R^2$ und $R^3$ unabhängig voneinander Alkyl, Arylalkyl, Heteroaryl oder Hydroxy bedeuten (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Alkylthio, Hydroxy, Nitro, Trifluormethoxy, Dialkylamino oder Cyano sind oder 2 benachbarte Positionen der Phenylgruppe durch $-O-(CH_2)_{1\ oder\ 2}-O-$verbunden sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

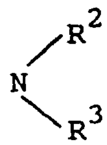

einen Ring der allgemeinen Formel

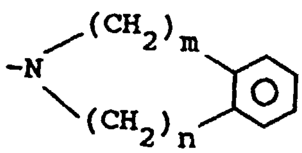 oder 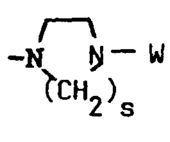

bedeuten, worin m und n je 0, 1, 2 oder 3 sind, wobei deren Summe 2, 3, 4 oder 5 ergibt, s 2 oder 3 ist,

W die Gruppe

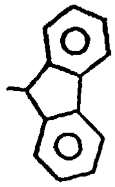 , 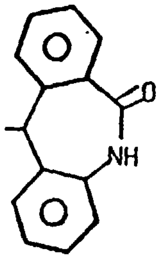 , 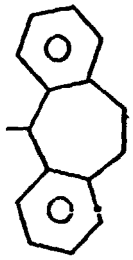

$(CH_2)_{0-2}$-Aryl, $CH(Aryl)_2$, Cyclopentyl, $(CH_2)_{0-2}$-Cyclohexyl, Pyridyl oder

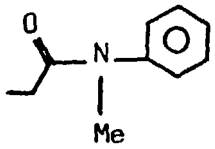

ist, (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl, Cyano, Hydroxy, Nitro oder Alkylthio sind oder 2 benachbarte Positionen der Phenylgruppe durch -O-$(CH_2)_{1-2}$-O-verbunden sind und Alkyl 1 bis 3 C-Atome enthält).

**2.** Verbindung nach Anspruch 1, worin

$R^1$ Vinyl, Aryl, Heteroaryl, Aralkyl, Heteroaralkyl, Arylvinyl, Heteroarylvinyl, Aryloxyalkyl, Arylalkyloxy, Di(arylalkyl)-aminoalkyl oder Arylalkylaminoalkyl (worin Aryl für Phenyl, 1-, 2- öder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L-Aminoprolin ($Pro(NH_2)$) wie beispielsweise 3-Aminoprolin ($Pro(3NH_2)$) und 4-Aminoprolin ($Pro(4NH_2)$), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D-oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), $Tpr(O_2)$ oder $Met(O_2)$, bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$A^2$ eine lipophile Aminosäure ist, die eine Phenyl-, 1-, 2- oder 3-fach substituierte Phenyl-, Heteroaryl-, Cyclohexyl- oder Cyclopentylgruppe oder eine Mono- oder Di-$C_{1-3}$-alkylaminogruppe enthält, und diese Ringgruppe bzw. Aminogruppe durch eine 1- bis 8-gliedrige Kette vom Backbone der Aminosäure getrennt ist, (wobei die Substituenten der Phenylgruppe unabhängig voneinander Halogen, Trihalogenomethyl, Alkoxy, Alkyl, Cyano oder 1-Pyrrolidinyl sind und die Kette wie in Anspruch 1 definiert ist) oder $A^2$ Leu, Ile, Nle, Val, Met
oder eine der Gruppen

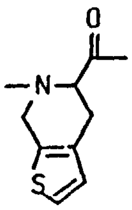
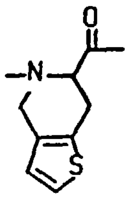
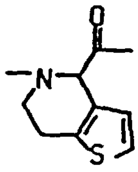
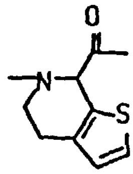

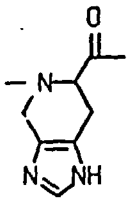
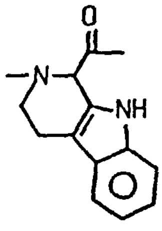
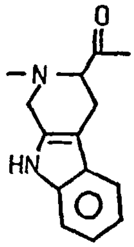

und

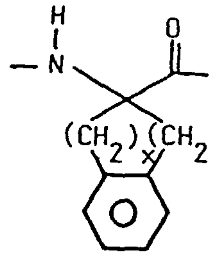

(worin x und y unabhängig voneinander 1 oder 2 sind) ist;

$R^2$ und $R^3$ unabhängig voneinander Alkyl, Arylalkyl, Heteroaryl oder Hydroxy bedeuten (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, Pyridyl, Pyrrolyl, Imidazolyl oder Thienyl steht; und die Alkyl- bzw. Alkoxygruppe 1 bis 3 Kohlenstoffatome enthält) oder die Gruppe

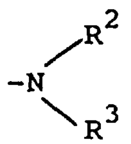

einen Ring der allgemeinen Formel

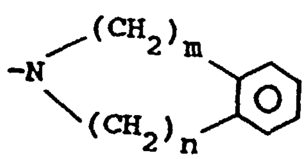

oder

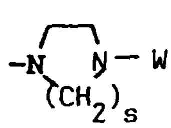

bedeuten, worin m, n und s wie in Anspruch 1 definiert sind, und

W die Gruppe

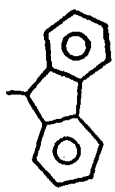

,

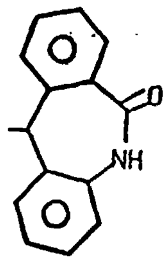

$-(CH_2)_{0-2}$-Aryl, $CH(Aryl)_2$, Cyclopentyl oder $(CH_2)_{0-2}$-Cyclohexyl ist, (worin Aryl für Phenyl, 1, 2 oder 3-fach substituiertes Phenyl oder Naphthyl steht; die Substituenten der Phenylgruppe unabhängig voneinander Halogen, Trihalogenomethyl, Alkoxy, Alkyl oder Cyano sind).

**3.** Verbindung nach Anspruch 1 oder 2, worin

$R^1$ Aryl, Heteroaryl, Aralkyl, Heteroaralkyl, Aryloxyalkyl, Arylalkyloxy, Di(arylalkyl)-aminoalkyl (worin Aryl für Phenyl oder 1- oder 2-fach substituiertes Phenyl steht; die Substituenten der Phenylgruppe unabhängig voneinander Halogen oder Alkoxy sind; Heteroaryl für Indolyl, in Stellung 1 durch Alkyl oder Benzyl substituiertes Indolyl oder Pyridyl steht; und die Alkyl- bzw. Alkoxygruppe 1 bis 3 Kohlenstoffatome enthält) bedeutet, insbesondere

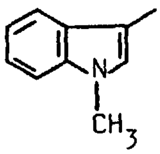

oder

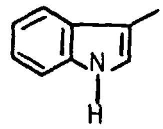

,

vorzugsweise

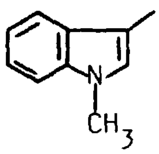

.

**4.** Verbindung nach Anspruch 1, 2 oder 3, worin $A^1$ eine Aminosäure ist, die eine oder 2 polare funktionelle Gruppe(n) in der Seitenkette trägt, wie OH, COOH, $NH_2$, Guanidin, $CONH_2$, SH.

**5.** Verbindung nach Anspruch 4, worin die funktionelle Gruppe in der Seitenkette von $A^1$ OH ist.

**6.** Verbindung nach Anspruch 1, 2 oder 3, worin $A^1$ Ser, Thr, Trp(For) oder Tyr ist.

**7.** Verbindung nach Anspruch 1, 2 oder 3, worin $A^1$ Pro oder 4-Hydroxyprolin ist.

**8.** Verbindung nach Anspruch 7, worin $A^1$ 4-Hydroxyprolin mit 2-S-Konfiguration ist, insbesondere

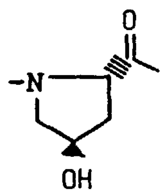

**9.** Verbindung nach einem der Ansprüche 1-8, worin $A^2$ für eine acyclische oder cyclische Aminosäure wie (O-Benzyl)Ser, (O-subst. Benzyl)Ser, (O-Benzyl)Thr, Cyclohexylalanin, Homophenylalanin, 3-(1-Pyrrolyl)-alanin, 3-(2,5-Dimethyl-1-pyrrolyl)alanin, 3-(1-Indolyl)alanin, 2-Amino-4-(1-Pyrrolyl)-buttersäure, 2-Amino-5-(1-Pyrrolyl)valeriansäure, 2-Amino-6-(1-Pyrrolyl)capronsäure, Leu, Lys(Z), 3-(2-Thienyl)alanin, 3-(3-Benzothienyl)alanin, 3-(1-Isoindolinonyl)alanin, (O-Benzyl)Asp, (O-Benzyl)Glu, Trp, (N-Me)Trp, His, 3-(2-Thiazolyl)-alanin, 3-Dimethylaminoalanin, -(O-Methyl)Tyr oder 2-Naphthylalanin,

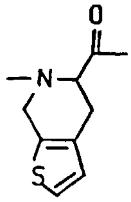
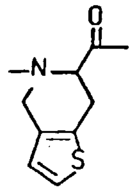
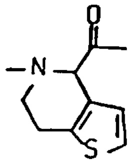
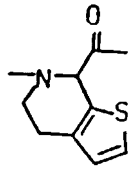

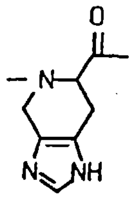
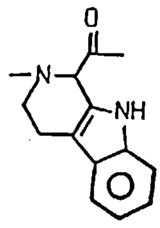
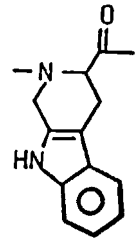

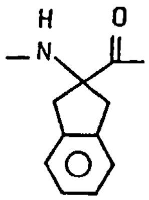
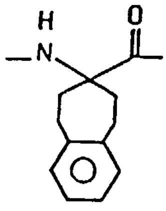
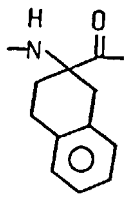
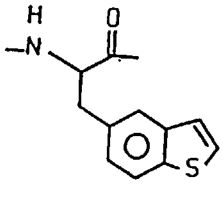

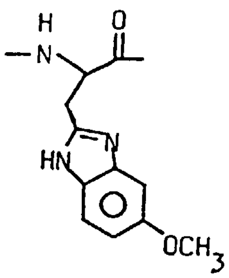
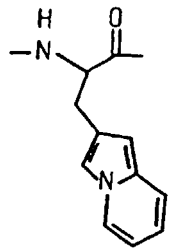
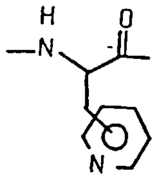

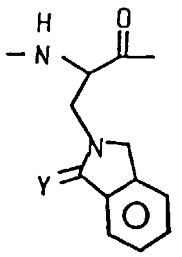
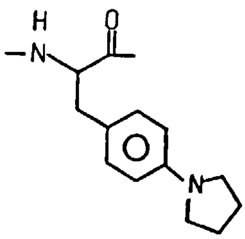
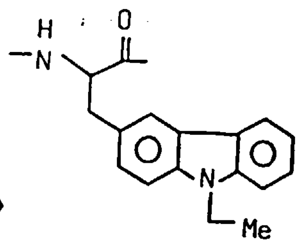

Y = $H_2$ oder O

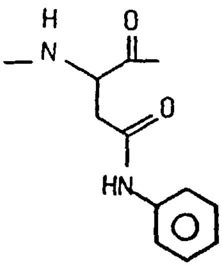
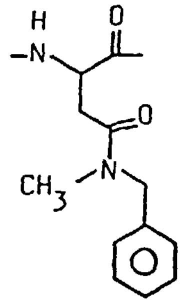
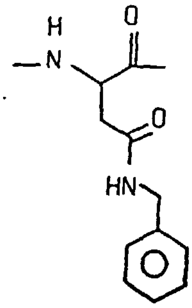

wobei die in den Aminosäuren enthaltenen Phenylgruppen 1-, 2- oder 3-fach substituiert sein können und die Substituenten unabhängig voneinander Halogen, Trihalogenomethyl, Alkoxy, Alkyl oder Cyano sind, die Alkyl- bzw. Alkoxygruppe 1 bis 3 Kohlenstoffatome enthält, und
wobei die angegebenen Aminosäuren vorzugsweise in S-Konfiguration vorliegen.

**10.** Verbindung nach Anspruch 9, worin

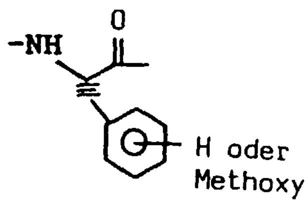
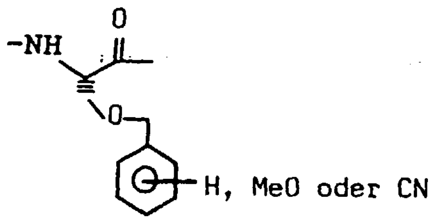

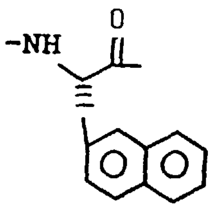

oder

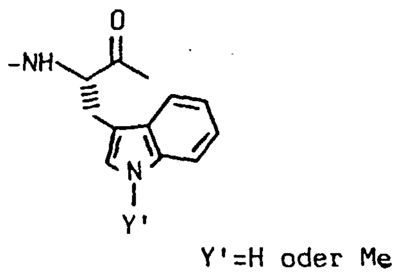

ist.

**11.** Verbindung nach einem der Ansprüche 1 bis 10, worin $R^2$ und $R^3$ unabhängig voneinander Methyl, Benzyl, Phenethyl (wobei die darin enthaltenen Phenylgruppen durch eine oder zwei Methoxygruppen substituiert sind) oder Pyridylmethyl ist.

**12.** Verbindung nach Anspruch 11, worin $R^2$ Methyl ist und $R^3$ Benzyl oder Alkoxybenzyl ist.

**13.** Verbindung nach Anspruch 12, worin $R^3$ Alkoxybenzyl, vorzugsweise 2-Methoxybenzyl ist.

**14.** Verbindung nach einem der Ansprüche 1 bis 10, worin die Gruppe

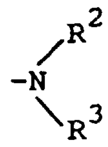

einen Ring

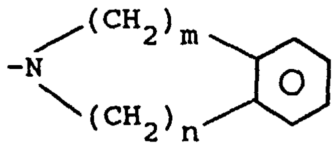

bedeutet, worin m 1 ist und n 1 oder 2.

**15.** Verbindung nach einem der Ansprüche 1 bis 10, worin die Gruppe

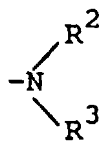

einen Ring

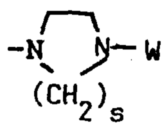

bedeutet, worin s 2 oder 3 ist (vorzugsweise 2) und W wie in Anspruch 1 definiert ist.

**16.** Verbindung nach Anspruch 15, worin W

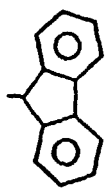

,

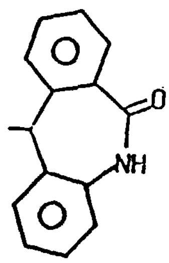

Cyclohexyl, Phenyl oder $CH(Phenyl)_2$ ist, worin die Phenylgruppen substituiert sind.

**17.** Verbindung nach Anspruch 16, worin wenn W Phenyl ist, dieses monosubstituiert ist durch Halogen, Alkoxy, Alkyl, Cyano, Hydroxy, Nitro oder Alkylthio.

**18.** Verbindung nach Anspruch 17, worin der Substituent der Phenylgruppe Methoxy, Chlor, Methyl, Ethyl, Cyano, Hydroxy, Nitro oder Methylthio, vorzugsweise Methoxy, Chlor, Methyl, Cyano oder Methylthio ist.

**19.** Verbindung nach Anspruch 17 oder 18, worin der Substituent der Phenylgruppe in Position 2 ist.

**20.** Verbindung nach Anspruch 16, worin wenn W die Gruppe -CH(Phenyl)$_2$ ist, die Phenylgruppe je durch ein Halogen substituiert sind, vorzugsweise durch Fluor.

**21.** Verbindung nach Anspruch 16 oder 20, worin in der -CH(Phenyl)$_2$-gruppe die beiden Phenylgruppen identisch substituiert sind, vorzugsweise in p-Stellung.

**22.** Verbindung nach Anspruch 1 oder 2, worin $R^4$ Wasserstoff, Methyl oder Phenyl, insbesondere Wasserstoff oder Methyl ist.

**23.** Verfahren zur Herstellung einer Verbindung nach einem der Ansprüche 1 bis 22 oder deren Salze, dadurch gekennzeichnet, daß man nach bekannten Methoden schrittweise die jeweiligen Aminosäuren, beziehungsweise Peptidderivatteilsequenzen, Carbonsäuren und Amine kondensiert und die so erhaltene Verbindung in freier Form oder in Form des gewünschten Salzes isoliert.

**24.** Pharmazeutische Zubereitung enthaltend eine Verbindung nach einem der Ansprüche 1 bis 22.

**25.** Verwendung einer Verbindung nach einem der Ansprüche 1 bis 22 zur Therapie von und zur Vorbeugung gegenüber Neurokinin-vermittelten Krankheiten.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 99 10 0929

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| P,X | WO 92 22569 A (FUJISAWA PHARMACEUTICAL CO) 23. Dezember 1992 (1992-12-23) * Ansprüche * --- | 1-14, 22-25 | C07K5/06 C07K5/02 |
| X | EP 0 443 132 A (FUJISAWA PHARMACEUTICAL CO) 28. August 1991 (1991-08-28) * Seite 15 - Seite 24 * --- | 1-14, 22-25 | |
| X | EP 0 394 989 A (FUJISAWA PHARMACEUTICAL CO) 31. Oktober 1990 (1990-10-31) * Seite 26 - Seite 69 * --- | 1-14, 22-25 | |
| X | EP 0 482 539 A (FUJISAWA PHARMACEUTICAL CO) 29. April 1992 (1992-04-29) * Seite 23 - Seite 36 * --- | 1-14, 22-25 | |
| X | EP 0 333 174 A (FUJISAWA PHARMACEUTICAL CO) 20. September 1989 (1989-09-20) * Zusammenfassung; Ansprüche * --- | 1-3, 10-14, 22-25 | |
| | -/-- | | RECHERCHIERTE SACHGEBIETE (Int.Cl.6) |
| | | | C07K |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Obwohl der Anspruch 25 sich auf ein Verfahren zur Behandlung des menschlichen/tierischen Körpers bezieht (Artikel 52(4) EPÜ), wurde die Recherche durchgeführt und gründete sich auf die angeführten Wirkungen der Verbindung/Zusammensetzung.

| Recherchenort | Abschlußdatum der Recherche | Prüfer |
|---|---|---|
| DEN HAAG | 24. September 1999 | Cervigni, S |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 99 10 0929

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | WO 91 12266 A (FUJISAWA PHARMACEUTICAL CO) 22. August 1991 (1991-08-22)<br><br>* Zusammenfassung; Ansprüche *<br>----- | 1-3, 10-14, 22-25 | |
| | | | RECHERCHIERTE SACHGEBIETE (Int.Cl.6) |

EPO FORM 1503 03.82 (P04C12)

## ANHANG ZUM EUROPÄISCHEN RECHERCHENBERICHT ÜBER DIE EUROPÄISCHE PATENTANMELDUNG NR. EP 99 10 0929

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-09-1999

| Im Recherchenbericht angeführtes Patentdokument | Datum der Veröffentlichung | Mitglied(er) der Patentfamilie | Datum der Veröffentlichung |
|---|---|---|---|
| WO 9222569 A | 23-12-1992 | EP 0590152 A | 06-04-1994 |
| | | JP 7503701 T | 20-04-1995 |
| EP 0443132 A | 28-08-1991 | AT 98651 T | 15-01-1994 |
| | | AU 640185 B | 19-08-1993 |
| | | AU 6801090 A | 27-06-1991 |
| | | CA 2032864 A | 23-06-1991 |
| | | CN 1064080 A,B | 02-09-1992 |
| | | CN 1159949 A | 24-09-1997 |
| | | DE 69005286 D | 27-01-1994 |
| | | DE 69005286 T | 21-04-1994 |
| | | DK 443132 T | 24-01-1994 |
| | | ES 2060910 T | 01-12-1994 |
| | | FI 906204 A,B, | 23-06-1991 |
| | | HK 18696 A | 09-02-1996 |
| | | HU 9500376 A | 28-08-1995 |
| | | IE 64570 B | 23-08-1995 |
| | | JP 2560919 B | 04-12-1996 |
| | | JP 4210996 A | 03-08-1992 |
| | | NO 177535 B | 26-06-1995 |
| | | PT 96324 A,B | 30-09-1991 |
| | | RU 2055078 C | 27-02-1996 |
| | | US 5468731 A | 21-11-1995 |
| EP 0394989 A | 31-10-1990 | AT 115961 T | 15-01-1995 |
| | | CA 2015359 A | 28-10-1990 |
| | | DE 69015244 D | 02-02-1995 |
| | | DE 69015244 T | 04-05-1995 |
| | | JP 3027399 A | 05-02-1991 |
| | | US 5164372 A | 17-11-1992 |
| EP 0482539 A | 29-04-1992 | AT 146480 T | 15-01-1997 |
| | | AU 647534 B | 24-03-1994 |
| | | AU 8592591 A | 30-04-1992 |
| | | CA 2054097 A | 25-04-1992 |
| | | CN 1060848 A,B | 06-05-1992 |
| | | CN 1148503 A | 30-04-1997 |
| | | DE 69123697 D | 30-01-1997 |
| | | DK 482539 T | 06-01-1997 |
| | | ES 2095283 T | 16-02-1997 |
| | | FI 914961 A | 25-04-1992 |
| | | GR 3022137 T | 31-03-1997 |
| | | HU 9500392 A | 28-09-1995 |
| | | JP 4297492 A | 21-10-1992 |
| | | PT 99317 A,B | 30-09-1992 |
| | | RU 2073683 C | 20-02-1997 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 99 10 0929

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-09-1999

| Im Recherchenbericht angeführtes Patentdokument | Datum der Veröffentlichung | Mitglied(er) der Patentfamilie | Datum der Veröffentlichung |
|---|---|---|---|
| EP 0482539 A | | US 5633232 A | 27-05-1997 |
| | | US 5420297 A | 30-05-1995 |
| EP 0333174 A | 20-09-1989 | AT 137763 T | 15-05-1996 |
| | | AU 3132489 A | 21-09-1989 |
| | | CA 1329444 A | 10-05-1994 |
| | | CN 1037156 A | 15-11-1989 |
| | | DE 68926403 D | 13-06-1996 |
| | | DE 68926403 T | 17-10-1996 |
| | | DK 126389 A | 17-09-1989 |
| | | FI 891176 A | 17-09-1989 |
| | | JP 1287095 A | 17-11-1989 |
| | | US 5187156 A | 16-02-1993 |
| WO 9112266 A | 22-08-1991 | EP 0515681 A | 02-12-1992 |
| | | US 5321032 A | 14-06-1994 |

EPO FORM P0461

Für nähere Einzelheiten zu diesem Anhang : siehe Amtsblatt des Europäischen Patentamts, Nr.12/82